# EUROPÄISCHE PATENTSCHRIFT

(11) **EP 3 513 168 B1**
(45) Veröffentlichungstag und Bekanntmachung des Hinweises auf die Patenterteilung: **05.08.2020**
(21) Anmeldenummer: 17764803.7
(22) Anmeldetag: 07.09.2017
(51) Int. Cl.: G01N 33/32, G01N 21/03, G01N 15/00, G01N 21/17, G01N 21/47, G01N 21/25, G01J 3/46, G01N 1/20, G01N 21/53, G01N 21/05

(54) **SENSOR ZUR QUASI-SIMULTANEN MESSUNG DER TRANSMISSION UND/ODER VORWÄRTSSTREUUNG UND/ODER REMISSION UND ZUR SIMULTANEN MESSUNG DER TRANSMISSION UND VORWÄRTSSTREUUNG, ODER TRANSMISSION UND REMISSION EINER FLÜSSIGEN PROBE**
SENSOR FOR SEQUENTIAL MEASUREMENT OF TRANSMISSION AND/OR FORWARD SCATTER AND/OR REMISSION OR SIMULTANEOUS MEASUREMENT OF TRANSMISSION AND FORWARD SCATTER, OR TRANSMISSION AND REMISSION OF A SAMPLE
CAPTEUR POUR LA MESURE SEQUENTIELLE DE L'ÉMISSION ET/OU DE LA DIFFUSION VERS L'AVANT ET/OU DE LA LUMINANCE DE RÉFLEXION OU POUR LA MESURE SIMULTANÉE DE LA TRANSMISSION ET DE LA DIFFUSION VERS L'AVANT OU DE LA TRANSMISSION ET DE LA LUMINANCE DE RÉFLEXION D'UN ÉCHANTILLON

(30) Priorität: 13.09.2016 EP 16188515
(43) Veröffentlichungstag der Anmeldung: 24.07.2019
(73) Patentinhaber: BASF Coatings GmbH, 48165 Münster (DE)
(72) Erfinder: JOCH, Andreas, 48165 Münster (DE); SCHAEFER, Michael, 67122 Altrip (DE); PEIXOTO, Carlos Arthur Leaes, 09715-030 Sao Bernardo do Campo (BR); ETTMUELLER, Juergen, 67056 Ludiwgshafen (DE); ZIEGLER, Stefan, 67056 Ludwigshafen (DE); MOONEN, Pieter, 48165 Münster (DE)
(74) Vertreter: BASF IP Association
(86) Internationale Anmeldenummer: PCT/EP2017/072427
(87) Internationale Veröffentlichungsnummer: WO 2018/050527

(56) Entgegenhaltungen:
- WO-A1-2009/065613
- DE-A1- 2 361 752
- GB-A- 796 745
- US-A1- 2008 273 204
- US-B1- 6 288 783

## Beschreibung

Die vorliegende Erfindung betrifft einen Sensor zur quasi-simultanen Messung der Transmission und/oder Vorwärtsstreuung und/oder Remission, und zursimultanen Messung der Transmission und Vorwärtsstreuung, oder Transmission und Remission einer flüssigen Probe, ein Verfahren zur quasi-simultanen Messung der Transmission und/oder Vorwärtsstreuung und/oder Remission, und zur simultanen Messung der Transmission und Vorwärtsstreuung, oder Transmission und Remission einer flüssigen Probe mit einem erfindungsgemäßen Sensor, und die Verwendung des erfindungsgemäßen Sensors zur quasi-simultanen Messung der Transmission und/oder Vorwärtsstreuung und/oder Remission, und zur simultanen Messung der Transmission und Vorwärtsstreuung, oder Transmission und Remission einer flüssigen Probe zur Bestimmung der Farbeigenschaften von Anstrichmitteln wie Lacken und Farben, Pasten und Pigmenten oder ihren Verdünnungen.

Das Entstehen eines Farbeindrucks ist physikalisch ein komplexer Vorgang, daher sollen die verwendeten Begriffe zunächst erläutert werden.

Grundsätzlich zu unterscheiden sind selbstleuchtende Objekte (zum Beispiel Lampen, Bildschirme, Leuchtfelder) und Objekte, die zur Betrachtung beleuchtet werden müssen. Dabei wird das Objekt entweder durchstrahlt und das durchtretende Licht betrachtet (Fachbegriff *Transmission*) oder im Auflicht bestrahlt, und das reflektierte Licht betrachtet (Fachbegriff *Remission*). "Farbig" nennt man Präparationen, die einen Teil des sichtbaren Lichtes absorbieren.

Als *Vorwärtsstreuung* werden im Allgemeinen Streuprozesse bezeichnet, bei denen es zu einer Ablenkung des Lichts in Vorwärtsrichtung kommt. Die Vorwärtsstreuung unterscheidet sich also unter anderem von der Transmission durch eine Ablenkung über einen Streuwinkel θ, wobei der Streuwinkel im Allgemeinen als der Winkel definiert ist, um den das gestreute Licht nach Interaktion mit einem Teilchen beziehungsweise einem Partikel abgelenkt wird. Als *Vorwärtsstreuung* (VS) werden im Sinne der Erfindung also Streuprozesse bezeichnet, bei denen es zu einer Ablenkung des Lichts in Vorwärtsrichtung im Streuwinkelbereich zwischen >0° und < 90° kommt und welche nicht von der Transmission erfasst werden. Die Vorwärtsstreuung erlaubt im Allgemeinen die Bestimmung der Größe und Isotropie von Teilchen oder Partikeln.

Von der Vorwärtsstreuung zu unterscheiden und hier lediglich zur Vollständigkeit aufgeführt ist die Quasi-Rückstreuung, bei der Licht in Folge einer diffusen Lichtausbreitung im dispersen Medium wieder in die Einstrahlrichtung, also in einem Streuwinkel von 180°, zurückgestreut wird. Diese einwinklige Rückstreumessung/ Quasi-Rückstreumessung beinhaltet nahezu keine Information zu Größe und Isotropie von Teilchen oder Partikeln.

Anschaulich sind die Begriffe "transparent" und "diffus streuend". Als bekannte Objekte kann man hier einerseits als Beispiele für "transparent" farblose oder gefärbte klare Folien oder Scheiben und andererseits als Beispiele für "diffus streuend" opake Folien (zum Beispiel Papier) oder Milchglas aufführen.

Färbende Präparationen unterscheiden sich ebenfalls nach diesem Gesichtspunkt, man redet zum Beispiel von deckenden oder transparenten Farben. Letztere benötigen einen streuenden, am besten weißen Untergrund, um die Farbe sichtbar werden zu lassen, also zum Beispiel Papier, Kunststofffolie oder Textilmaterial. Deckende Präparationen erzeugen eine farbige Oberfläche ohne Durchscheinen des Untergrundes (zum Beispiel Ölfarben, Automobillacke, Wandfarben) und sind entweder aus Mischungen von streuenden Pigmenten (Weißpigmente wie zum Beispiel TiO₂) und absorbierenden Bestandteilen (nicht oder wenig streuende Bunt-Pigmente und/ oder lösliche absorbierende Komponenten) zusammengesetzt, oder aus Pigmenten, die sowohl streuen als auch absorbieren.

Bei der industriellen Herstellung von farbigen Präparationen erwartet der Kunde, dass das nach einer Spezifikation gelieferte Produkt nach der Anwendung immer die gleiche Farbe erzeugt.

Die Farbe im Sinne von Remission und das Deckvermögen sind also Bestandteil der Produktspezifikation. Vereinfacht gesagt muss das Anstrichmittel, in der dem Fachmann üblichen Schichtdicke aufgetragen, auf schwarzem und weißem Untergrund die gleiche Farbe erzielen.

Es ist bei der Betrachtung von Farben erforderlich, den Begriff der Farbmessung zu erläutern. Es gibt mehrere technische Möglichkeiten dies durchzuführen. Die dabei anfallenden Ergebnisse sind im Prinzip übertragbar und vergleichbar, wenn auch nicht immer von gleichwertiger Detailauflösung. Die Farbmessung ist über die DIN-Norm 5033 definierbar. Üblich ist die Messung im Wellenlängenbereich des sichtbaren Lichtes von 380 nm bis 780 nm, wobei gelegentlich auch ein kleinerer Wellenlängenbereich betrachtet wird.

Alle Farbmessungen orientieren sich an der durchschnittlichen menschlichen Fähigkeit, direkt nebeneinanderliegende homogene Farbflächen zu unterscheiden. Das menschliche Auge kann nur 3 Farbreize, nämlich Rot, Grün und Blau unterscheiden. Diese Empfindlichkeitsbereiche werden in der Technik X, Y und Z genannt, und sind nicht ganz deckungsgleich mit den Spektren der additiven Farbmischung mit R, G und B wie zum Beispiel bei Fernsehern und Computerbildschirmen.

Es sind grundsätzlich zwei Lösungen für die Farbmessung gebräuchlich, einerseits Detektoren mit Filtern, andererseits Systeme mit einem wellenlängendispersiven Element im Strahlengang, welches das aufgespaltene Spektrum auf eine Diodenzeile oder CCD-Zeile abbildet.
Für die Variante mit Filtern und Detektoren gibt es folgende naheliegende Ausführungen:
- 3 Bereiche mit X, Y, Z beziehnugsweise R, G, B Filtern und 3 Detektoren
- n Bereiche mit n schmalbandigen Filtern und n Detektoren

Für die Variante mit wellenlängendispersivem Element werden typischerweise Varianten mit 128, 256, 512, 1024 oder mehr Detektionsbereichen verwendet. Eine geringere benötigte Wellenlängenauflösung wird durch Zusammenrechnen benachbarter Kanäle realisiert. Diese Methoden zur Datenreduktion sind dem Fachmann bekannt.

Grundsätzlich möglich ist auch der umgekehrte Weg, der Empfang erfolgt mit einem breitbandigen Detektor, der im gesamten untersuchten Wellenlängenbereich empfindlich ist, und die Wellenlängenselektion erfolgt beleuchtungsseitig, indem nacheinander 3 oder mehr verschiedene Lichtquellen analog zu den vorstehend beschriebenen Wellenlängenbereichen eingeschaltet werden oder eine Weißlichtquelle mit einem wellenlängendispersivem Element gekoppelt wird.

Eine Farbmessung, die nur 3 Wellenlängenbereiche abfragt, ist oft schon ausreichend. Die genauesten Messungen bieten Messgeräte, die das gesamte sichtbare Spektrum in sehr enge Bereiche, zum Beispiel 2 - 20 nm Wellenlänge, unterteilen.

Bei der wissenschaftlichen Beschreibung von farbigen Präparationen haben sich die Begriffe Streuung (S) und Absorption (K oder A) zur Charakterisierung von farbgebenden Substanzen etabliert, als Referenz für alle Theorien und Formeln, die die Farbe von farbigen Schichten beschreiben, sei hier auf die dem Fachmann bekannte Kubelka-Munk Theorie verwiesen, die die von einer mit Weißlicht bestrahlten Schicht gegebener Stärke (d) reflektierte (Remission P(λ)) oder transmittierte (Transmission T(λ)) Intensität bei der Beobachtungswellenlänge λ berechnet.

Es ist bisher nur Stand der Technik, diese Berechnungen für isotrop wirkende Pigmente und Farbmittel ausführen zu können. Wissenschaftliche Ansätze, dies auch für beliebige Farbmittel mit zum Beispiel Effektpigmenten berechnen zu können, sind bisher nicht öffentlich verfügbar.

Berechnungen, aus bekannten konventionellen Pigmenteigenschaften die Eigenschaften des resultierenden gemischten Produktes zu bestimmen, sind veröffentlicht, und werden in der Praxis auch eingesetzt.

Ebenfalls Stand der Technik sind Methoden, aus Messungen von Mischungen des Produktes mit bekannten Referenzmaterialien (Weiß- und Schwarzpasten) die Streu- und Absorptions-Eigenschaften des Produktes zu bestimmen.

Alternativ ist es auch möglich, ausgehend vom Typ-Muster des Produktes, bekannte Mengen der Produktkomponenten hinzu zu dosieren und die Auswirkungen in Form von Konzentrationsreihen zu erfassen. Auf Basis solcher Daten ist es dann möglich, eine Abweichung der Farbe einer Produktcharge als Abweichung der Rezeptur zu interpretieren und entsprechend zu korrigieren.

Mit verschiedenen mathematischen Inversionsmethoden kann man auch Berechnungsverfahren entwickeln, um aus den gemessenen Eigenschaften der Pigmentpräparation die Eigenschaften und/oder Konzentrationen der einzelnen Komponenten der Präparation zu bestimmen.

Obwohl sich die Eigenschaften infinitesimal dünner Schichten von Pigmenten für alle Farbmittel beschreiben lassen (zum Beispiel Mie-Theorie, beziehungsweise diskrete Dipol-Approximation) ist die Berechnung der Mehrfachstreuung von Schichten praxisnaher Dicke (also nicht infinitesimal dünner) nicht Stand der Technik.

Bei der Herstellung von Anstrichmitteln aller Art ist es jederzeit möglich eine Probe zu nehmen, damit eine Probetafel herzustellen, diese zu trocknen und gegebenenfalls noch thermisch zu behandeln (einbrennen) und die dabei entstehende Farbe zu messen. Aus den Abweichungen zur vereinbarten Musterfarbe "Typ" kann man dann Korrekturschritte bestimmen. Dabei sind der erzielte Farbton und das erzielte Deckvermögen zwei unabhängige, jeweils einzustellende Spezifikationen. Um die Problematik zu erläutern, sei eine Farbe, die aus der Mischung von Weißpigment und einem Farbpigment besteht, betrachtet. Ist das Mischungsverhältnis aus Weiß und Farbe abweichend, wird die Farbe zu blass oder zu intensiv beziehungsweise dunkel. Dem Fachmann ist bekannt, dass nach Kubelka-Munk die Farbe nur vom Verhältnis K/S, der absorbierenden zur streuenden Komponente, abhängt, aber wenn von beidem zu wenig in der Präparation enthalten ist, wird das erforderliche Deckvermögen nicht erreicht (beim Anwender schimmert der Untergrund dann durch).

Deshalb wird zur Sicherheit oft mehr Pigment als nötig zu den Formulierungen hinzugegeben, beziehungsweise dosiert, um auch bei unvermeidlichen Schwankungen der Qualität der Eingangsprodukte, des Herstellungs- und des Applikationsprozesses auf der sicheren Seite zu liegen um somit die Kundenspezifikationen vollumfänglich zu erfüllen.

Gerade bei Anstrichmitteln im niedrigen Preissegment (zum Beispiel Bautenfarben) ist dies jedoch von Nachteil, da man damit teure Pigmente höher dosiert als für die gewünschte Farbwirkung nötig ist und zusätzlich die sonstigen Eigenschaften des Produktes unter Umständen nachteilig beeinflusst.

Ebenfalls sind die Bestimmung sowie die Qualitäts- beziehungsweise Beschaffenheitsüberwachung von Effektpigmenten oder flüssigen Effektpigmentpräparationen, beispielsweise von Al- und Mica-Pigmenten wie diese auf dem Gebiet der Lacke und Farben angewendet werden, im Allgemeinen schwierig und aufwendig. Für eine vollständige Charakterisierung müssen die Partikelgröße, die winkelabhängige Farbe der Partikel und der allgemeine Farbeindruck erfasst werden.

Erforderlich ist daher ein Messinstrument, welches es erlaubt die farblichen Eigenschaften wie Farbort, Deckvermögen und Farbstärke (siehe zum Beispiel Römpp Lexikon, Georg Thieme Verlag) flüssiger Proben, das heißt einzelner Komponenten oder einer fertigen Produktmischung, also von Anstrichmitteln wie Lacken und Farben, Pasten und Pigmenten mit hoher Messgenauigkeit möglichst einfach und schnell zu bestimmen.

US2008/0273204 A1 betrifft eine Vorrichtung und eine Methode zur Messung der Transmission und Remission einer flüssigen Probe, insbesondere einer Farbe oder einer anderen opaken Flüssigkeit, umfassend eine referenzierte Lichtquelle **1**, einen Strahlteiler **18** (*"switch"*), eine durchströmbare Messzelle **11** mit einem einstellbaren Messspalt ([0110]), mindestens eine Empfangsoptik und mindestens einen Detektor sowie einen Lock-in Verstärker **5**, der das Signal-Rausch-Verhältnis deutlich verbessern soll. Ein Strahlteiler **18** kann dabei den von der Lichtquelle **1** über eine Glasfaser **10** stammenden Lichtstrom aufteilen, wodurch eine simultane oder separate Messung der Remission und Transmission möglich ist. Durch Verschieben der Position des Strahlteilers **18** sind Beleuchtungswinkel und damit auch der Remissionswinkel einstellbar ([0126]). Ebenfalls kann die Vorwärtsstreuung ("*second transmitted electromagnetic radiation*") unter einem Winkel von 45° erfasst werden ([0127]).

EP 0 472 899 A1 betrifft eine photometrische Vorrichtung zur Messung des Dämpfungsgrades bei der Lichtausbreitung in dispersen Systemen, umfassend eine durchströmbare Küvette (1), eine Lichtquelle (4), mindestens eine Beleuchtungsoptik in Form von Lichtwellenleitern (LWL), und mindestens eine Empfangsoptik in Form von Lichtwellenleitern (LWL), wobei durch Auswahl der Faserzahl und Anordnung in den Lichtwellenleitern sowie die Art der Einkopplung in die Küvette, beispielsweise unter einem Winkel von 45° oder durch eine 400 bis 700 µm starke aufgesteckte Glasplatte (Gp) für die Remissionsmessung, simultan Transmission und Remission oder Transmission und Quasi-Rückstreuung erfasst werden können.

Aus der WO2005/003740 ist ein Remissionssensor zur Messung einer flüssigen Probe bekannt, die aufgebaut ist aus
a) einer optischen Einheit (A), die eine Lichtquelle (Aa) in Form einer Lampe, und eine Faseroptik umfassend Lichtwellenleiter (Ab), wobei mindestens ein Lichtwellenleiter ein Referenzleiter ist, umfasst, und
b) einer Probenanalyseeinheit (B), die ein Messfenster (Ba) und eine Probenanalysezelle (Bb) umfasst, wobei auf einer Seite des einen Messfensters die optische Einheit angeordnet ist und auf der anderen Seite desselben Messfensters die Probenanalysezelle angeordnet ist, indem diese so an das Messfenster angepresst ist, dass ein Spalt zwischen Messfenster und Probenanalysezelle gebildet wird, den eine zu messende Probe in Form einer flüssigen Pigmentpräparation durchqueren muss, wobei bei der Durchquerung des Spalts eine erhebliche Scherung der Probe erfolgt, und
c) einer System-Kontrolleinheit (C) umfassend Detektoren (Ca) zur Aufnahme von Messdaten und ein daran angeschlossenes Auswertegerät (Cb),
   wobei mindestens eine Lichtwellenleiterverbindung von der Lichtquelle (Aa) zu dem Messfenster (Ba) und von dem Messfenster (Ba) weiter zum Detektor (Ca) geführt wird zur Erzeugung eines Messsignals (Remission des Produkts), und mindestens eine Referenzleiterverbindung direkt von der Lichtquelle (Aa) zum Detektor (Ca) oder vom Messfenster (Ba) zum Detektor (Ca) geführt wird zur Erzeugung eines Referenzsignals (interner Reflex).

Zusätzlich zum obig beschriebenen Remissionssensor (WO2005/003740) offenbart die WO2005/062022 eine dreidimensionale Strömungszelle zur Ausrichtung von nicht-isometrischen Partikeln in einer flüssigen Probe in zwei Achsen und ihre Verwendung mit einem Remissionssensor. Grundsätzlich wird auch die Möglichkeit genannt mit der photometrischen Messeinrichtung Transmission oder Quasi-Rückstreuung zu messen.

US 6,040,913 und WO 00/45152 betreffen ein Verfahren zur Bestimmung der Streuungseffizienz eines Weißpigments, worin die Lichtstreuungseffizienz mit der Performance in der Endanwendung korreliert, umfassend die Herstellung einer verdünnten Suspension eines Pigments in einem flüssigen Medium mit einer bekannten Konzentration des Pigments in der Suspension, Messung der totalen Transmission (T) der Suspension bei einer Wellenlänge von mindestens 600 nm, Umwandlung der Transmissionsmessung (T) in die optische Dichte (OD) durch die Gleichung: OD = -log (T) und Teilen der optischen Dichte durch die Konzentration des Pigments in der Suspension, um die Lichtstreuungsintensität des Pigments zu bestimmen.

Die WO 2013/173401 betrifft ein Verfahren zur Messung einer oder mehrerer Eigenschaften einer Flüssigkeit, umfassend:
Herstellung eines dünnen Films einer Probe der Flüssigkeit mit einer vorbestimmten Filmdicke im Bereich von 0,05 mm bis 2 mm unter Verwendung eines Dünnschichtbauelements umfassend:
C1:
   (a) eine kreisförmige planare Scheibe (101) enthaltend eine erste Oberfläche (101a) und eine zweite Scheibenoberfläche (101b) auf den gegenüberliegenden Seiten der kreisförmigen planaren Scheibe, wobei die kreisförmige planare Scheibe mit einem rotierenden Schaft (120) verbunden ist, verbunden mit einer rotationssymmetrischen Achse (110) von der kreisförmigen planaren Scheibe, senkrecht zu den Scheibenoberflächen, um eine Rotation der kreisförmigen planaren Scheibe zu ermöglichen;
   (b) einen Bauelement-Rahmen (121), der die kreisförmige planare Scheibe und den Rotationsschaft positioniert;
   (c) ein Dickenkontrollelement (125) enthaltend eine Film einfassende Kante (125a) verbunden mit einem Rückkehrkanal für Flüssigkeit (125b) und mindestens einem Rahmen-Verbindungsglied (125c), der die Film einfassende Kante (125a) und den Rückkehrkanal für Flüssigkeit (125b) mit dem Bauelement-Rahmen (121) verbindet, wobei das Rahmen-Verbindungsglied in Beziehung zu dem Bauelement-Rahmen beweglich ist; und
   (d) ein Bewegungselement (130) verbunden mit dem Rotationsschaft zur Ermöglichung der Rotation des Rotationsschafts (120) und ein Bewegungskontrollelement (131) zur Kontrolle der Rotationsgeschwindigkeit, Rotationsrichtung oder einer Kombination von beidem des Rotationselements,
      worin das Dickenkontrollelement (125) auf der ersten Oberflächenseite (101a) der kreisförmigen planaren Scheibe (101) positioniert ist, die Film einfassende Kante (125a) im Wesentlichen parallel zu der ersten Oberfläche positioniert ist und die Film einfassende Kante (125a) mit der kreisförmigen planaren Scheibe überlappt, wobei sie einen Bereich von 50% bis 99% des Radius der kreisförmigen planaren Scheibe(101) bedeckt;
      worin der Abstand (127) zwischen der Film einfassenden Kante (125a) und der ersten Oberfläche (101a) im Bereich von 0,05 bis 5 mm liegt und über das Rahmen-Verbindungsglied einstellbar ist; und worin die vorbestimmte Filmdicke durch diesen Abstand (127) und durch die Rotationsgeschwindigkeit und Richtung kontrolliert wird;
C2:
   Messung des dünnen Films mit einer oder mehreren Messvorrichtungen, um Probendaten zu erhalten; und
C3:
   Einstellung der einen oder mehreren Eigenschaften basierend auf den Probendaten.

EP 0 932 829 betrifft ein Analysesystem zur Analyse der physikalischen Eigenschaften von Lacken, Pigmentpasten oder ähnlichen Systemen, das aus einer Vorrichtung zur Ausbildung eines Films der Lacke, Pigmentpasten und ähnlichen Systemen mit einer spezifischen Dicke, einer Lichtquelle zum Bestrahlen des zu untersuchenden Lacks oder der zu untersuchenden Pigmentpaste oder ähnlichen Systemen aufgebaut ist, wobei eine Wechselwirkung zwischen dem Licht und dem Lack, der Pigmentpaste oder ähnlichen Systemen auftritt, wobei ein Messsignal erzeugt wird; und einer Vorrichtung zur Aufnahme des Messsignals sowie einem mit der Vorrichtung zur Aufnahme des Messsignals verbundenen Detektor.

WO 2009/065613 A1 betrifft eine Vorrichtung zur Ermittlung der Partikelkonzentration, der Partikelgröße, der mittleren Partikelgröße und der Partikelgrößenverteilung von Partikeln einer dispersen Phase sowie dessen Trübung durch Messen der Transmission und/oder durch Messen des Streulichts eines Messstrahls nach zurücklegen einer definierten Messstrecke innerhalb des dispersen Systems, wobei Ablagerungen an der Messstrahlemissions- und Messstrahlkollektorfläche weitgehend vermieden werden.

US 6,288,783 B1 betrifft ein Fluidanalysesystem zum Analysieren einer spezifizierten physikalischen Eigenschaft eines Fluides und ein Verfahren für dieses, wobei das System ein Filmbildungsmittel aufweist, um einen Fluidfilm mit einer bestimmten Dicke zu bilden, eine Filmbestrahlungseinrichtung, die geeignet ist, den Film mit elektromagnetischer Strahlung zu bestrahlen, um eine Wechselwirkungsstrahlung zu erzeugen, einen Rezeptor zum Empfangen der Wechselwirkungsstrahlung und einen dem Rezeptor zugeordneten Detektor zum Erfassen der Wechselwirkungsstrahlung. Des Weiteren ist mindestens eine der gegenüberliegenden Fluidkontaktflächen für elektromagnetische Strahlung durchlässig und weist das System eine Oberflächenreinigungsanlage auf.

Die Messanordnungen aus dem Stand der Technik besitzen eine unzureichende Messgenauigkeit, Geschwindigkeit und Möglichkeit zur Durchführung mehrerer hochgenauer spektrometrischer Untersuchungen an einer flüssigen Probe.

Im bisherigen Stand der Technik ungelöst ist die zuverlässige Erfassung von spektrometrischen Messdaten zur Ermittlung der farblichen Eigenschaften wie Farbort, Deckvermögen und Farbstärke, beziehungsweise Absorption (K oder A) und Streuung (S), flüssiger Proben, das heißt einzelner Komponenten oder einer fertigen Produktmischung, also von Anstrichmitteln wie Lacken und Farben, Pasten und Pigmenten mit hoher Genauigkeit und hinreichender Geschwindigkeit. Insbesondere fehlt es an einer Messvorrichtung, die eine vollständige Charakterisierung von winkelabhängigen Partikeln wie beispielsweise Titandioxid oder Al- und Mica-Effektpigmenten erlaubt. Ebenfalls ungelöst im Stand der Technik ist eine kontinuierliche Erfassung spektrometrischer Messdaten mit hoher Genauigkeit und hinreichender Geschwindigkeit an einer flüssigen Probe, insbesondere einer strömenden flüssigen Probe.

### Lösung der Aufgabe

Zur Lösung der obengenannten Aufgabenstellung wurde ein Sensor zur quasi-simultanen Messung der Transmission und/oder Vorwärtsstreuung und/oder Remission, und zur simultanen Messung der Transmission und Vorwärtsstreuung, oder Transmission und Remission einer flüssigen Probe gefunden, nach Anspruch 1.

Der neue Sensor zur quasi-simultanen Messung der Transmission und/oder Vorwärtsstreuung und/oder Remission, und zur simultanen Messung der Transmission und Vorwärtsstreuung, oder Transmission und Remission einer flüssigen Probe, wird in der Folge auch als erfindungsgemäßer Sensor bezeichnet. Bevorzugte Ausführungsformen des erfindungsgemäßen Sensors gehen aus der folgenden Beschreibung sowie den Unteransprüchen hervor.

Gegenstand der vorliegenden Erfindung ist auch ein Verfahren zur quasi-simultanen Messung der Transmission und/oder Vorwärtsstreuung und/oder Remission, und zur simultanen Messung der Transmission und Vorwärtsstreuung, oder Transmission und Remission einer flüssigen Probe mit dem erfindungsgemäßen Sensor in einer diskontinuierlichen und kontinuierlichen Fahrweise, nach Anspruch 11. Zudem betrifft die vorliegende Erfindung die Verwendung des erfindungsgemäßen Sensors zur Bestimmung der Farbeigenschaften von Anstrichmitteln wie Lacken und Farben, Pasten und Pigmenten oder ihren Verdünnungen, nach Anspruch 14.

### Beschreibung der Erfindung

### "Quasi-simultan"

Unter dem Ausdruck "quasi-simultan" ist im Sinne der vorliegenden Erfindung ein sequentielles Erfassen von Transmission und/oder Vorwärtsstreuung und/oder Remission zu verstehen, das innerhalb von 1 ms bis 10 s erfolgt. Es wurde gefunden, dass ein sequentielles Erfassen von Transmission und/oder Vorwärtsstreuung und/oder Remission innerhalb von 1 ms bis 10 s keine nachteiligen Auswirkungen auf die Messgenauigkeit hat.

### "Simultan"

Erfolgt ein simultanes - das heißt gleichzeitiges - Erfassen von Transmission und Vorwärtsstreuung, oder Transmission und Remission, wird dies bevorzugt wie nachstehend durchgeführt:
Das Messsystem kann so modifiziert werden, dass nur eine Lichtquelle (LQ) auf 90° (senkrecht) verwendet wird, um eine simultane Messung der Transmission und Vorwärtsstreuung unter Verzicht auf Remission zu ermöglichen, oder um eine simultane Messung der Transmission (180°) und Remission unter Verzicht auf Vorwärtsstreuung und Verzicht auf den Remissionswinkel von 110° zum Glanzwinkel zu ermöglichen.

### "Der erfindungsgemäße Sensor"

Der erfindungsgemäße Sensor eignet sich zur quasi-simultanen Messung der Transmission und/oder Vorwärtsstreuung und/oder Remission und zur simultanen Messung der Transmission und Vorwärtsstreuung, oder Transmission und Remission einer flüssigen Probe.

Die folgenden Messvarianten sind somit von dem erfindungsgemäßen Sensor umfasst:

### Quasi-simultane Messvarianten:

a) Transmission und Vorwärtsstreuung
b) Transmission und Remission
c) Transmission und Vorwärtsstreuung und Remission
d) Vorwärtsstreuung und Remission

### Simultane Messvarianten:

e) Transmission und Vorwärtsstreuung
f) Transmission und Remission

Die Wortlaute "quasi-simultan" und "simultan" sind im Sinne der Erfindung demnach so auszulegen, dass mindestens zwei der drei aufgeführten Messarten Transmission und/oder Vorwärtsstreuung und/oder Remission durchzuführen sind, wobei mit dem erfindungsgemäßen Sensor offensichtlich

auch die Einzelmessungen der Transmission, Vorwärtsstreuung, oder Remission durchgeführt werden können. Bevorzugt werden jedoch quasi-simultan mindestens zwei Messungen ausgewählt aus Transmission, Vorwärtsstreuung und Remission in Kombination durchgeführt. Simultan werden immer zwei Messungen in Kombination durchgeführt.

Vorstehend und im Folgenden ist unter "REM" Remission, unter "VS" Vorwärtsstreuung und unter "TM" Transmission zu verstehen.

Unter "TM/VS" ist im Sinne der vorliegenden Anmeldung "TM" und/oder "VS", also Transmission und/oder Vorwärtsstreuung zu verstehen.

### "Probe"

Unter einer Probe ist im Sinne der vorliegenden Erfindung eine flüssige Zusammensetzung aus einzelnen Komponenten oder einer fertigen Produktmischung, insbesondere farbiger Pigmentpräparation, zu verstehen. Der Ausdruck "farbig" umfasst dabei neben beliebigen Farben auch "schwarz" und "weiß".

Flüssige Zusammensetzungen einzelner Komponenten oder fertige Produktmischungen, insbesondere farbige Pigmentpräparationen, liegen zum Beispiel in Form von Anstrichfarben, Lacken oder Pigmentpasten beziehungsweise als stabile Verdünnungen von Anstrichfarben, Lacken oder Pigmentpasten vor.

Unter stabilen Verdünnungen von Anstrichfarben, Lacken oder Pigmentpasten sind Lösungen oder Dispersionen von Anstrichfarben, Lacken oder Pigmentpasten zu verstehen, die sich nicht entmischen, ausflocken oder aggregieren. Geeignete Lösungs- und Dispergiermittel sind dem Fachmann bekannt. Beispiele sind Wasser, organische Lösungsmittel, Klarlacke oder Bindemittel.

Mit Hilfe des erfindungsgemäßen Sensors können quasi-simultan Transmission und/oder Vorwärtsstreuung und/oder Remission und simultan Transmission und Vorwärtsstreuung, oder Transmission und Remission einer flüssigen Probe in Form einer Schicht bestimmt werden.

Die Probe in Form einer Schicht ist im Sinne der Erfindung eine zwischen zwei Messfenstern (MF1) und (MF2) vorliegende, bevorzugt strömende, flüssige Pigmentpräparation oder ihre entsprechende Verdünnung, die vorstehend und nachstehend als Probe bezeichnet wird.

Für bestimmte Produkte ist eine Verdünnung der Probe, zum Beispiel zur Verbesserung der Fließfähigkeit und gegebenenfalls zur Anpassung des Messbereichs, vorteilhaft. Dadurch kann zum Beispiel eine unnötig hohe Dosierung teurer Pigmente, die die Eigenschaften der Proben nachteilig beeinflussen kann, vermieden werden.

### Zeichnungen

Im Folgenden wird die vorliegende Erfindung Anhand von Zeichnungen erläutert. Die in den Zeichnungen verwendeten Abkürzungen haben dabei nachstehende Bedeutung:
- TM: Transmission
- REM: Remission
- VS: Vorwärtsstreuung

- LQ: Lichtquelle (REM, TM, VS)
- BO: Beleuchtungsoptik (REM, TM, VS)
- STS: Strahlteilersystem
- RO: Referenzoptik (REM, TM, VS; REF)

- DET: Detektor (REM, TM, VS; REF)
- EO: Empfangsoptik (REM, TM, VS)

- ST: Stößel
- DS(2): Dichtsystem Stößel
- P(DS2): Pumpe Dichtsystem Stößel
- V(DS2): Vorlage Dichtsystem Stößel
- AK: Aktor (Positioniersystem)
- C-AK: Controller Aktor (Controller Positioniersystem)
- POS: Positionsmesssystem
- C-Pos: Controller Positionsmesssystem

- MZ: Messzelle
- MF: Messfenster
- MS: Messspalt
- DS(1): Dichtsystem Messzelle
- GP: Grundplatte
- K: Kanal, gebildet aus dem Raum zwischen Messzelle (MZ), Messfenster (MF1) und vorgefahrenem Stößel (ST), zur ungehinderten Passage einer flüssigen Probe durch die Messzelle (MZ).

- MG: Messgerät
- P(P): Pumpe Probe
- V(P): Vorlage Probe
- PC(P): Druckregler Probe

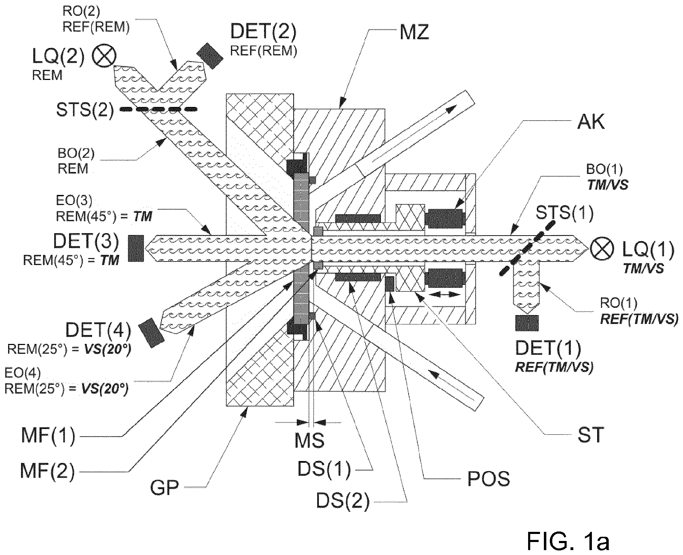

In den **Figuren 1a** bis **1d** sind beispielhafte Anordnungen einer oder mehreren Lichtquellen (LQ), einer oder mehreren Beleuchtungsoptiken (BO), mindestens einer Messzelle (MZ), mindestens einer Empfangsoptik (EO)(TM/VS/REM) und mindestens eines Detektors (DET)(TM/VS/REM) zur Messung von durch Transmission erzeugten Transmissionssignalen, zur Messung von durch Vorwärtsstreuung erzeugten Vorwärtsstreusignalen beziehungsweise zur Messung von durch Remission erzeugten Remissionssignalen in dem erfindungsgemäßen Sensor dargestellt.
**Figur 1a** zeigt eine beispielhafte Anordnung des erfindungsgemäßen Sensors mit zwei REM Detektoren (DET)(3,4) und ihren entsprechenden Empfangsoptiken (EO)(3,4) unter einem Winkel von 25° beziehungsweise 45° zum Glanzwinkel der Lichtquelle (LQ)(2)(REM), von denen (DET)(3) auch als Transmissionsdetektor unter einem Winkel von 180° zur Lichtquelle (LQ)(1)(TM/VS) und (DET)(4) als einer der möglichen Vorwärtsstreu-Detektoren unter einem Winkel von 20° zur Lichtquelle (LQ)(1)(TM/VS) genutzt werden kann, sowie die im Strahlengang den Lichtquellen (LQ)(1,2) nachgeschalteten Beleuchtungsoptiken (BO)(1,2). Zudem sind die bevorzugt in Verbindung mit den Lichtquellen (LQ)(1)(TM/VS) und (LQ)(2)(REM) einzusetzenden Referenzdetektoren (DET)(1)(REF TM/VS) beziehungsweise (DET)(2)(REF REM) inklusive den dann zum Einsatz kommenden Strahlteilersystemen (STS)(1,2) und Referenzoptiken (RO)(1)(TM/VS) beziehungsweise (RO)(2)(REM) abgebildet.
   In der beispielhaften Anordnung des erfindungsgemäßen Sensors in **Figur** 1a sind also die Lichtquelle (LQ)(REM), die Beleuchtungsoptik (BO)(REM), die mindestens eine Empfangsoptik (EO)(TM/VS/REM), und der mindestens eine Detektor (DET)(TM/VS/REM) auf der Seite des Messfensters (MF1), die Lichtquelle (LQ)(TM/VS) und die Beleuchtungsoptik (BO)(TM/VS) auf der Seite des gegenüberliegenden Messfensters (MF2), angeordnet.

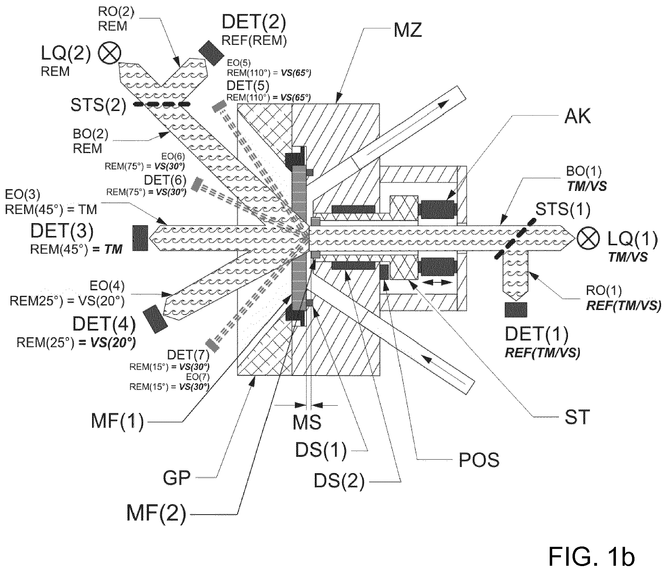

**Figur 1b** zeigt eine bevorzugte Anordnung des erfindungsgemäßen Sensors mit fünf REM Detektoren (DET)(3, 4, 5, 6, 7) und den jeweiligen Empfangsoptiken (EO)(3, 4, 5, 6, 7) unter Winkeln von 15°, 25°, 45°, 75°, und 110° zum Glanzwinkel der Lichtquelle (LQ)(2)(REM) von denen (DET)(3) auch als Transmissionsdetektor und (DET)(4, 5, 6, 7) als mögliche Vorwärtsstreu-Detektoren unter einem Winkel von 20°, 30° beziehungsweise 65° zur Lichtquelle (LQ)(1)(TM/VS) genutzt werden können, zwei Referenzdetektoren (DET)(1)(TM/VS) und (DET)(2)(REM) mit den jeweiligen Referenzoptiken (RO)(1)(TM/VS) und (RO)(2)(REM) sowie zwei Lichtquellen (LQ)(1)(TM/VS) und (LQ)(2)(REM).
   In der bevorzugten Anordnung des erfindungsgemäßen Sensors in **Figur 1b** sind also die Lichtquelle (LQ)(2)(REM), die Beleuchtungsoptik (BO)(2)(REM), die Empfangsoptiken (EO)(3, 4, 5, 6, 7)(TM/VS/REM), und die Detektoren (DET)(3, 4, 5, 6, 7)(TM/VS/REM) auf der Seite des Messfensters (MF1), die Lichtquelle (LQ)(1)(TM/VS) und die Beleuchtungsoptik (BO)(1)(TM/VS) auf der Seite des gegenüberliegenden Messfensters (MF2) angeordnet.

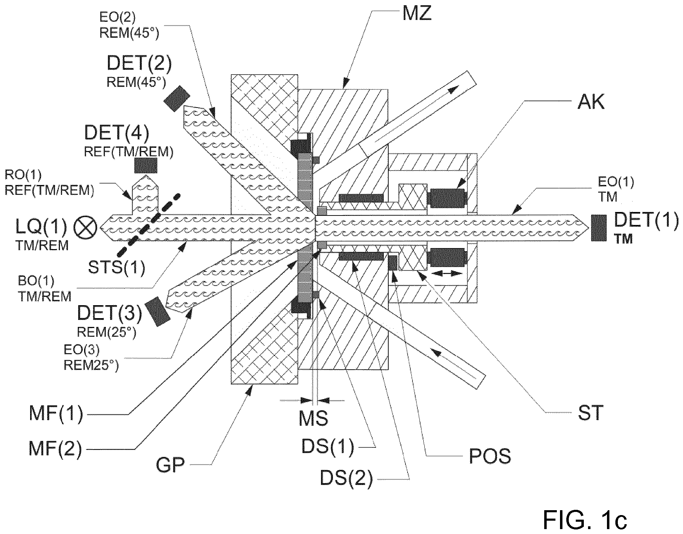

**Figur 1c** zeigt eine beispielhafte Anordnung mit nur einer Lichtquelle (LQ). Die eine Lichtquelle (LQ)(1)(TM/REM), zwei REM Detektoren (DET)(2,3)(REM) und ihre entsprechenden Empfangsoptiken (EO)(2,3) sind dabei an der Seite des Messfensters (MF1), ein TM Detektor (DET)(1)(TM) an dem der Lichtquelle (LQ)(1)(TM/REM) gegenüberliegenden Messfenster (MF2), angeordnet. Wird, wie bevorzugt, ein Referenzdetektor eingesetzt, befindet sich dieser Referenzdetektor (DET)(4)(TM/REM) zusammen mit der Referenzoptik (RO)(1) und das Strahlteilersystem (STS)(1) an der Seite des Messfensters (MF1).

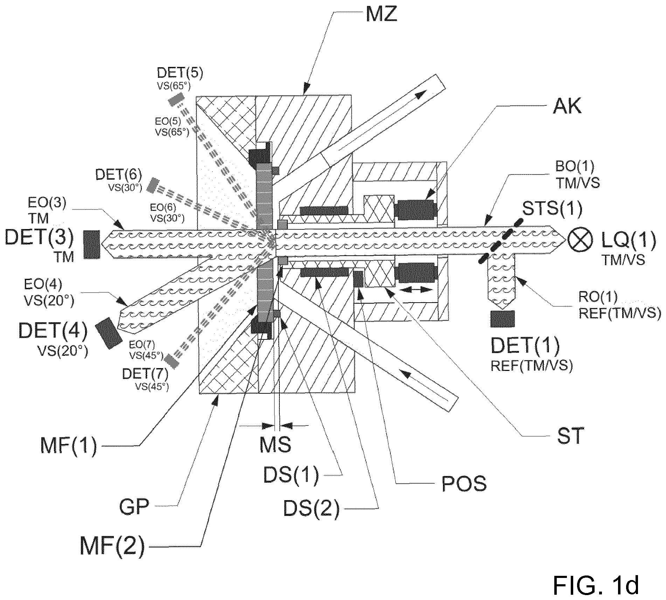

**Figur 1d** zeigt eine weitere beispielhafte Anordnung mit nur einer Lichtquelle (LQ)(1)(TM/VS), angeordnet an der Seite des Messfensters (MF2). An der Seite des Messfensters (MF1) sind vier VS Detektoren (DET)(4, 5, 6, 7)(VS) und ihre entsprechenden Empfangsoptiken (EO)(4, 5, 6, 7)(TM) unter einem Winkel von 20°, 30°, 45° und 65° zur Lichtquelle LQ(1)(TM/VS) und ein TM Detektor (DET)(3)(TM) unter einem Winkel von 180° zur Lichtquelle LQ(1)(TM/VS) angeordnet. Der bevorzugt einzusetzende Referenzdetektor (DET)(1)(TM/VS) und die entsprechende Referenzoptik (RO)(1) sowie das Strahlteilersystem (STS)(1) sind an der Seite des Messfensters (MF2) angeordnet.

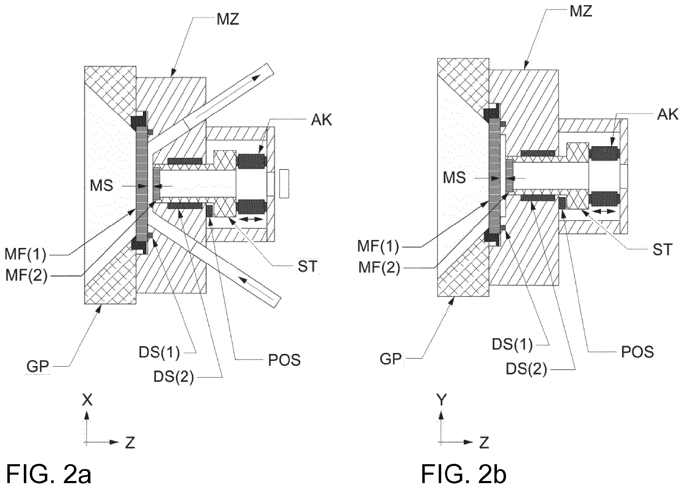

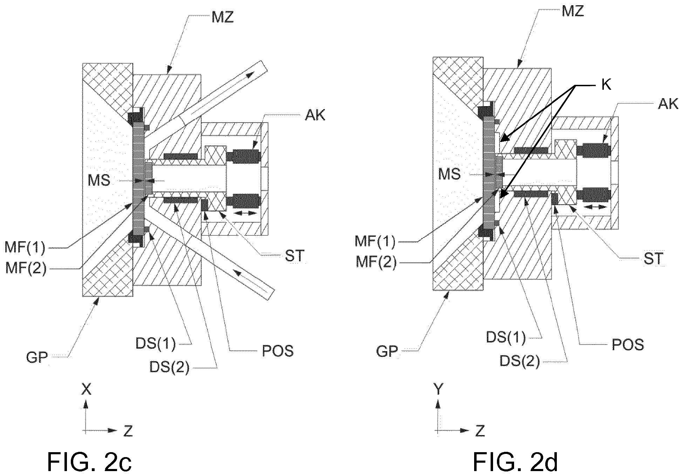

**Figur 2a** bis **Figur 2d** zeigen eine beispielhafte Anordnung einer bevorzugten Ausführungsform einer Messzelle (MZ), in der ein Messfenster (MF2) mit einem Stößel (ST) verbunden ist, wobei der Stößel (ST) axial beweglich ist über Aktoren (AK), wobei die Figuren 2a und 2c längs zur Strömungsachse und Figuren 2b und 2d quer zur Strömungsachse geschnitten sind.
**Figuren 2a** und **2b** zeigen zwei Perspektiven der beispielhaften Anordnung mit geöffnetem Messspalt (MS), also eine Anordnung in der ein Stößel (ST) weit zurückgezogen ist und der Abstand zwischen Messfenster (MF1) und (MF2) maximal ist.
**Figuren 2c** und **2d** zeigen zwei Perspektiven der beispielhaften Anordnung mit (nahezu) geschlossen Messspalt (MS); eine Anordnung also in die der Abstand zwischen Messfenster (MF1) und (MF2) durch vorschieben eines Stößels (ST) in die Messzelle (MZ) deutlich reduziert ist. Sie stellt somit eine mögliche Anordnung während der Messung einer Probe dar.
**Figur 2d** zeigt dabei deutlich, dass die Messzelle (MZ) durch das vorschieben des Stößels (ST) und damit des Messfenster (MF2), wodurch der Messspalt (MS) eingestellt wird, nicht völlig verschlossen ist, sondern dass der in die Messzelle (MZ) hineinragende Stößel (ST) mit der Wand der Messzelle und dem Messfenster (MF1) zwei Kanäle (K), in Strömungsrichtung links und rechts vom Stößel (ST), ausbildet, durch die ein Teil des in die Messzelle (MZ) hineingeleitete Probenstroms den Stößel (ST) seitlich umfließen kann und auf dieser Weise ungehindert (und nicht vermessen) die Messzelle (MZ) passiert.

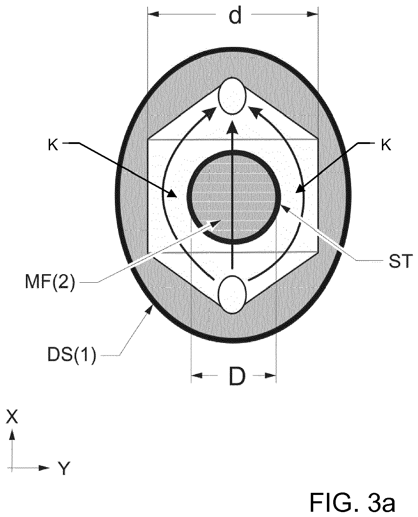

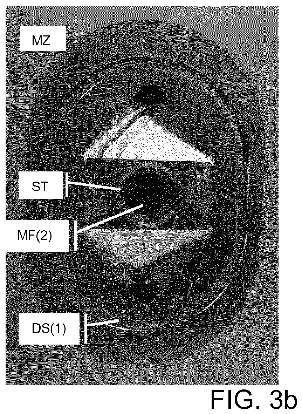

**Figur 3a** zeigt eine schematische Darstellung und **Figur 3b** ein Foto einer Seite einer realisierten Messzelle (MZ) mit zurückgezogenem Messfenster (MF2) und Stößel (ST), entsprechend der schematisch dargestellten Anordnung aus den Figuren 2a und 2b, worin noch mal verdeutlicht wird, wie ein Teil des Probenstroms ungehindert den Stößel (ST) über die in Strömungsrichtung links und rechts angeordneten Kanäle (K) umfließen kann, wenn dieser vorgefahren wird und der Durchmesser (D) des Messfensters (MF2) geringer ist als die Breite (d) der Grundfläche der Messzelle (MZ). Der andere Teil der Probe fließt, wie durch den geradlinigen Pfeil in Figur 3a dargestellt, während der Messung zwischen den Messfenstern (MF1) und (MF2) durch. Des Weiteren sind die Zu- und Abfuhrkanäle der Probe in die Messzelle (MZ) sowie eine Einkerbung, die beispielsweise mit einem abdichtenden Gummiring eine mögliche Ausführung eines Dichtsystems Messzelle (DS) darstellt, gezeigt.

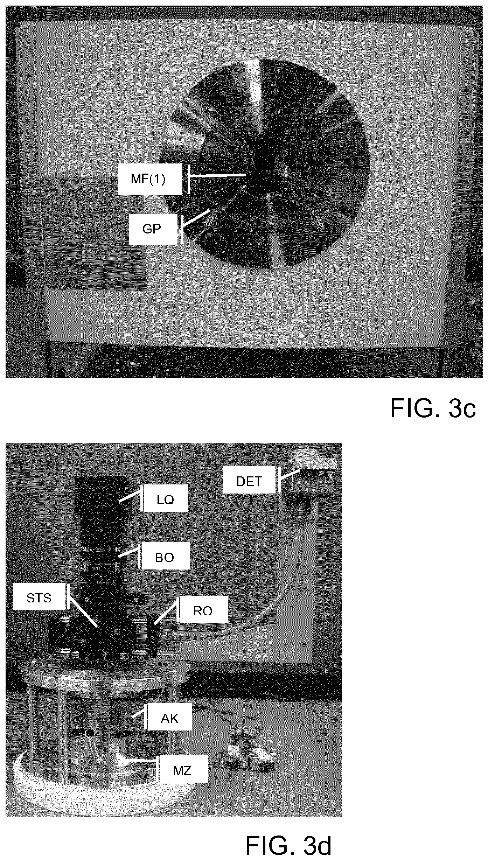

**Figur 3c** zeigt ein Foto der Gegenseite der in Figur 3b dargestellten Messzelle (MZ), in der ein Messfenster (MF1) auf eine Grundplatte (GP) montiert ist.
**Figur 3d** zeigt ein Foto einer bevorzugten Anordnung mit einer Lichtquelle (LQ), einer Beleuchtungsoptik (BO), eines Strahlteilersystems (STS), einer Referenzoptik (RO), eines Detektors (DET) und eines Aktors (AK) zum Antrieb eines Stößels (ST) (wobei ein weiterer Aktor (AK), das Positionsmesssystem (POS) und der Stößel (ST) verdeckt sind).

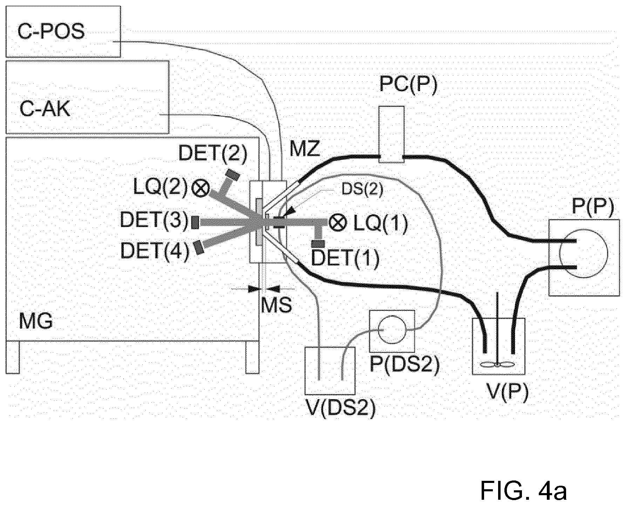

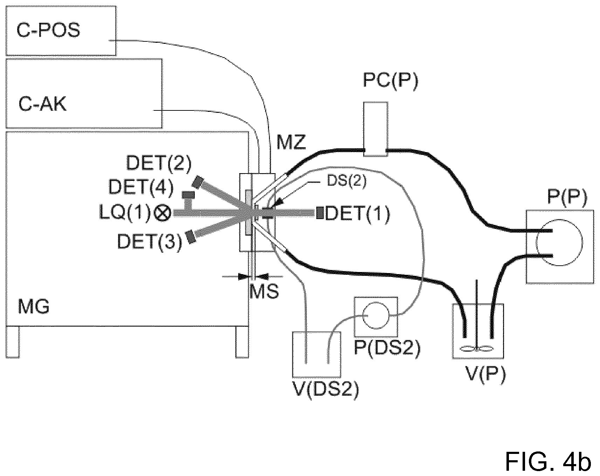

**Figuren 4a** und **4b** zeigen zwei beispielhafte Ausführungsformen eines Gesamtaufbaus des erfindungsgemäßen Sensors mit einer beispielhaften Ausführung einer Probenzufuhr und -abfuhr sowie ein Dichtsystem des Stößels (DS2).
**Figur 4a** entspricht dabei den optischen Aufbau beschrieben in Figur 1a, **Figur 4b** entspricht den optischen Aufbau beschrieben in Figur 1c.

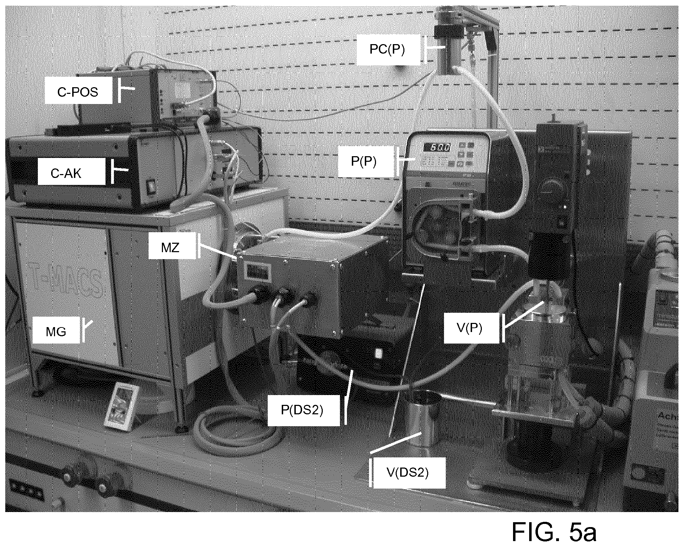

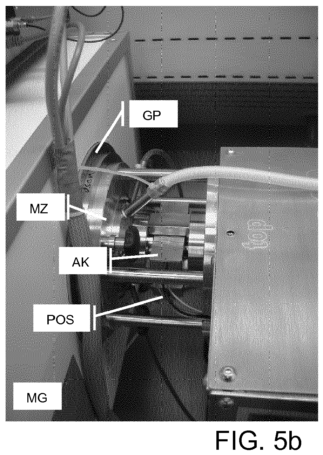

**Figur 5a** und **Figur 5b** zeigen Fotos eines realisierten Gesamtaufbaus entsprechend des optischen Aufbaus dargestellt in Figur 4a.

Die in den Zeichnungen dargestellten Elemente und in den Beschreibungen zu den Zeichnungen verwendeten Ausdrücke sowie geeignete Ausführungsformen davon sind, soweit sie in der vorliegenden Anmeldung nicht näher erläutert sind, dem Fachmann bekannt und üblicherweise in verfahrenstechnischen und optomechanischen Messaufbauten verwendete Elemente.

### Detaillierte Beschreibung der Erfindung

Im Folgenden werden die einzelnen Komponenten des erfindungsgemäßen Sensors a) bis e) beschrieben:

### a) eine oder mehrere Lichtquellen (LQ), bevorzugt zwei Lichtquellen (LQ)(TM/VS) und (LQ)(REM)).

### Physikalische Funktion:

Grundsätzlich geht jede einzelne Messung von einer Lichtquelle (LQ) aus, die Licht im benötigten Wellenlängenbereich mit der erforderlichen Intensität, Konstanz und Langlebigkeit zur Verfügung stellt.

Als Lichtquellen (LQ) sind sehr viele verschiedene Prinzipien denkbar, angefangen von Lampen mit Glühwendel, Gasentladungslampen, bis hin zu LEDs. Der Vorteil von LEDs ist die Möglichkeit, die Intensität durch Konstantstrombetrieb konstant zu halten und auf mechanische Shutter verzichten zu können (Strom aus = Licht aus). Auch die Verwendung von Lasern ist möglich.

Die Lichtquellen (LQ) sind somit bevorzugt unabhängig voneinander - für den Fall, dass mehrere Lichtquellen eingesetzt werden - ausgewählt aus der Gruppe bestehend aus LEDs, bevorzugt Weißlicht-LEDs, RGB-LEDs, Arrays von LEDs mit aneinandergrenzenden Wellenlängenbereichen; Lampen mit Glühwendel; Gasentladungslampen.

Die Lichtquellen (LQ) zeigen im Allgemeinen eine gute thermische und mechanische Stabilität, das heißt wenig Schwankungen im Kurzzeitbereich, keine große Veränderung im Langzeitbereich, lange Lebensdauer. Bevorzugt zeigen die Lichtquellen ein gleichmäßiges Spektrum, so dass alle Wellenlängen, die an der Probe gemessen werden sollen, mit einer ausreichenden Intensität am Detektor angelangen.

Bevorzugt können die Lichtquellen (LQ) beliebig oft ein- und ausgeschaltet werden, besonders bevorzugt mittels mechanischem Shutter, Schaltung der Spannungsversorgung der Lichtquelle und/oder optoelektrischem Shutter. Ganz besonders bevorzugt wird die Lichtquelle durch Schaltung der Spannungsversorgung ein- und ausgeschaltet.

Eine bevorzugte Ausführungsform der vorliegenden Anmeldung betrifft eine Anordnung, worin hinter der Lichtquelle ein Kompensationsfilter angeordnet ist. Dabei ist unter "hinter der Lichtquelle" zu verstehen, dass der Kompensationsfilter dem Verlauf des Lichtstrahls der Lichtquelle folgend nach der Lichtquelle angeordnet ist. Der in dieser Ausführungsform eingesetzte Kompensationsfilter linearisiert das Spektrum der Lichtquelle so, dass das Verhältnis zwischen höchster und niedrigster Intensität des von der Lichtquelle ausgestrahlten Lichts maximal 4, bevorzugt 3 bis 4, ist und nicht, was im Stand der Technik üblich ist, 10 bis 20 ist. Dies wird mit handelsüblichen Filtergläsern erreicht.

In einer weiteren bevorzugten Ausführungsform sind hinter der Lichtquelle (LQ) - bei Einsatz eines Kompensationsfilters zwischen Lichtquelle und Kompensationsfilter - ein IR-Sperrfilter, ein Kondensor und eine Streuscheibe angeordnet. Wiederum bedeutet "hinter der Lichtquelle" im Sinne der vorliegenden Anmeldung dem Lichtstrahl folgend nach der Lichtquelle. Der IR-Sperrfilter dient dazu, die Wärmebelastung, die durch die Lichtquelle auf die Probe, die Lichtwellenleiter, den Kompensationsfilter und andere Einheiten des Sensors einwirkt, zu reduzieren.

Der Kondensor dient dazu, dass Licht der Lichtquelle auf den Eingang der Optik, zum Beispiel Faseroptik, zu bündeln. Die Streuscheibe dient dazu, einen strukturfreien, gleichmäßigen Verlauf der Helligkeit des Lichts der Lichtquelle über dem Ort und dem Aperturwinkel der Lichtwellenleiter zu erreichen. Geeignete Ausführungen von Kondensoren und Streuscheiben, die für den erfindungsgemäßen Sensor geeignet sind, sind dem Fachmann bekannt.

Der erfindungsgemäß in einer Ausführungsform in die Lichtquelle integrierte Shutter ist zum Beispiel ein elektromechanischer Shutter, der die Beleuchtungsfaser vollständig verdunkeln kann. Die Abdunkelung der Lichtquelle dient der Messung des Dunkelstroms (elektrischer temperaturabhängiger Offset des Detektors und der dazugehörigen Verstärkerelektronik), der vom Signalmesswert bei eingeschalteter Lichtquelle subtrahiert werden muss. In der hier ganz besonders bevorzugten Form der Dunkelstrommessung wird die Lichtquelle ausgeschaltet. Die vom Spektrometer gemessene Lichtmenge wird durch das Auslesen zurückgesetzt, aber eventuell (je nach Hardware) nur zu etwa 99 %, so dass ein Rest der letzten Messung im Spektrometer verbleibt und die erste Dunkelstrommessung verfälscht. Ab der zweiten aufeinanderfolgenden Dunkelstrommessung ist der Restwert im Rahmen der Messgenauigkeit unerheblich.

### b) eine oder mehrere Beleuchtungsoptiken (BO), bevorzugt zwei Beleuchtungsoptiken (BO)(TM/VS) und (BO)(REM).

### Physikalische Funktion Beleuchtungsoptik (BO):

Das von der Lichtquelle ausgestrahlte Licht wird in einem bestimmten Raumwinkelbereich von einem optischen Aufbau aufgenommen und auf das Messobjekt (hier im Allgemeinen die Schicht einer flüssigen Probe, beispielsweise eine farbige Präparation) geleitet. Diesen Aufbau nennt man Beleuchtungsoptik.

Bevorzugt weist der Sensor zusätzlich zu der oder den Beleuchtungsoptiken (BO) eine oder mehrere Referenzoptiken (RO), bevorzugt (RO)(TM/VS) und (RO(REM), auf und zusätzlich zu dem oder den Detektoren (DET)(TM/VS/REM) mindestens einen Detektor, bevorzugt zwei Detektoren, als Referenzdetektoren (RDET), bevorzugt (RDET)(TM/VS) und (RDET)(REM).

### Physikalische Funktion Referenzoptik (RO), Referenzdetektor (RDET):

Um die Genauigkeit der Messung zu optimieren, kann ein Teil des von der Lichtquelle (LQ) ausgesandten Lichtes ausgekoppelt und mit einer separaten Optik (die Referenzoptik (RO)) zu einem Referenzdetektor (RDET) geführt werden.

Die Beleuchtungsoptik (BO) beleuchtet die Messfläche der Produktprobe mit einem bestimmten Raumwinkel. Möglich sind Beleuchtungen von verschiedenen Hauptwinkeln, in der "Trockenfarbmetrik" bekannt sind 0° und 45°, und mit verschiedenen Aperturwinkeln. Eine parallele Beleuchtung hat den Aperturwinkel ∼0°, bei der ebenfalls bekannten "diffusen" Beleuchtung gelangt das Licht aus allen Raumrichtungen zur Messfläche. Dabei geht es nicht um besser oder schlechter, sondern um eine gute Vergleichbarkeit der Nassmessung mit der Trockenmessung. In der wissenschaftlichen Literatur und bei einigen Herstellern wird auch die Verwendung von verschiedenen Beleuchtungsrichtungen diskutiert.

Die Beleuchtungsoptik (BO) kann im Allgemeinen mit Linsen und Blenden "konventionell" ausgeführt sein, aber auch faseroptische Elemente enthalten. Wichtig ist die Konstanz der Beleuchtungsintensität und der Lage des beleuchteten Flecks. Die für den Referenzdetektor (RDET) bereitgestellte Lichtmenge sollte passend sein, diesen im Simultanbetrieb mit dem Messdetektor auszusteuern, und immer ein konstantes Verhältnis zur Beleuchtungsintensität aufweisen. Die Referenzauskopplung kann bei faseroptischen Systemen durch eine im Einkopplungsstecker parallel mitgefasste Faser erfolgen oder bei konventionellen Systemen durch einen teildurchlässigen Spiegel im Strahlengang.

Somit sind die Beleuchtungsoptiken (BO) bevorzugt unabhängig voneinander als faseroptisches System und/oder als konventionelles System mit Linsen und Blenden ausgeführt.

Die Referenzoptik (RO), bevorzugt Referenzoptiken (RO)(TM/VS) und (RO)(REM), ist somit bei faseroptischen Systemen bevorzugt durch eine in einem Einkopplungsstecker parallel mitgefasste Faser ausgeführt und bei konventionellen Systemen bevorzugt durch einen teildurchlässigen Spiegel oder ähnlich wirkende optische Systeme, zum Beispiel Strahlteilerprisma, Glasplatte, im Strahlengang ausgeführt.

Die Lichtwellenleiter sind aus Glasfasern oder Kunststofffasern oder Flüssiglichtleitern, als Einzelfasern oder Faserbündel, aufgebaut. Bevorzugt sind Glasfasern mit 100, 200, 400, 600, 800 µm oder größerem Faserdurchmesser oder Faserbündel, zum Beispiel am Spektrometer, fest montiert. Besonders bevorzugt weist die als Referenzleiter eingesetzte Faser einen angepassten, bevorzugt kleineren Durchmesser auf als die übrigen Lichtwellenleiter, damit ein repräsentativer Teil des Lichtes der eingesetzten Lichtquelle (LQ), bevorzugt LED, im Wesentlichen direkt zum Referenzdetektor (RDET) gelangt und möglichst exakt gemessen wird.

Der Referenzleiter wird in einer bevorzugten Ausführungsform des erfindungsgemäßen Sensors über ein Dämpfungselement, das heißt ein präzises Abstandselement mit eingebauter Streuscheibe, um den vollen Aperturwinkel zu erhalten, geführt.

Die vorstehenden Ausführungen gelten ebenfalls für die unter d) erwähnte Empfangsoptik (EO).

### c) mindestens eine Messzelle (MZ), wobei die Messzelle (MZ) eine durchströmbare Zelle ist, die zwei gegenüberliegende parallel angeordnete Messfenster (MF1) und (MF2) aufweist, die so zueinander angeordnet sind, dass ein Messspalt (MS) zwischen den Messfenstern (MF1) und (MF2) gebildet wird, der von der zu messenden Probe ausgefüllt wird, wobei die Messfenster (MF1) und (MF2) einen definierten Abstand zueinander aufweisen der variabel einstellbar ist.

### Physikalische Funktion:

Die zu messende Schicht wird durch eine flüssige Probe gebildet, die in der Regel den durch zwei gegenüberliegende parallel angeordnete Messfenster (MF1) und (MF2) gebildeten Spalt (Messspalt (MS)) ausfüllt. Bevorzugt handelt es sich bei der Probe um eine strömende Probe, wobei der Strömungsweg der durch die zwei gegenüberliegenden parallel angeordneten Messfenster (MF1) und (MF2) gebildeter Messspalt (MS) ist.

Die Messzelle (MZ) ist eine durchströmbare Zelle, die zwei gegenüberliegende Messfenster (MF1) und (MF2) (für Remission, Transmission und Vorwärtsstreuung) in festem oder variablem, aber stets präzise gemessenem Abstand bereitstellt, zwischen denen sich die Probe, bevorzugt die strömende Probe, befindet.

Zum Transport der zu prüfenden Probe in einen Messspalt (MS) wird dieser geöffnet, das heißt der Abstand zwischen den beiden Messfenstern (MF1) und (MF2) wird vergrößert, bis die Schicht sich im Allgemeinen vollständig erneuert hat. Daraufhin wird der Messspalt (MS) zwischen den Messfenster (MF1) und (MF2) für die eigentliche Messung (wieder) auf die gewünschte Messposition verengt beziehungsweise der Abstand zwischen den beiden Messfenstern (MF1) und (MF2) reduziert, wobei die überschüssige Probe bevorzugt ungehindert über die Kanäle (K) entweichen kann. Die verbleibende Probe wird im Allgemeinen noch leicht geschert, was der Messung förderlich ist.

Der definierte Abstand der Messfenster (MF1) und (MF2) ist, im durchströmten Betrieb, variabel einstellbar, das heißt dass der Messspalt (MS) und damit auch die Schichtstärke der Probe, bevorzugt der strömenden Probe, gebildet durch den definierten Abstand der gegenüberliegenden parallel angeordneten Messfenstern (MF1) und (MF2), variiert werden kann, wobei der eingestellte definierte Abstand stets präzise bestimmbar ist. Ein, im durchströmten Betrieb, variabel einstellbarer aber stets präzise bestimmbarer Abstand kann benötigt werden, um den Messspalt (MS) beziehungsweise die Schichtstärke individuell, bevorzugt stufenlos, an die jeweilige Probe anzupassen, um auf diese Art, je nach Bedarf, eine Schichtstärke einzustellen bei der die Probe deckend, semitransparent oder transparent ist.

Der definierte Abstand der Messfenster (MF1) und (MF2) beträgt bevorzugt 1 bis 10000 µm, besonders bevorzugt 5 bis 5000 µm, ganz besonders bevorzugt 10 bis 500 µm.

Bevorzugt sind die Messfenster (MF1) und (MF2) jeweils Planplatten, unabhängig voneinander aufgebaut aus für den verwendeten Wellenlängenbereich transparentem Material mit hinreichender mechanischer und chemischer Widerstandsfähigkeit, zum Beispiel aus Kunststoff (zum Beispiel PS, PC, PET, PMMA), Glas (zum Beispiel BK7, BaK2, LaSF9, Borosilikatglas, Quarzglas), Quarz, Zirkonia, Halbedelstein (zum Beispiel Zirkon), oder Edelstein (zum Beispiel Topas, Saphir, Diamant). Bevorzugt besitzt das Messfenster (MF1) eine Dicke von 1 bis 12 mm, besonders bevorzugt 4 bis 10 mm, ganz besonders bevorzugt 6 bis 8 mm. Das Messfenster (MF1) weist im Allgemeinen einen Durchmesser von 10 bis 100 mm, bevorzugt 30 bis 70 mm auf.

Bevorzugt besitzt das Messfenster (MF2) eine Dicke von 1 bis 12 mm, besonders bevorzugt 1 bis 8 mm, ganz besonders bevorzugt 1 bis 4 mm. Das Messfenster (MF2) weist im Allgemeinen einen Durchmesser (D) von 5 bis 100 mm, bevorzugt 10 bis 30 mm auf.

Gemäß der Erfindung ist der definierte Abstand zwischen den Messfenstern (MF1) und (MF2) im durchströmten Betrieb variabel einstellbar, d.h. dass die strömende Probe nicht angehalten, unterbrochen, reduziert, oder andersartig angepasst werden muss, um den definierten Abstand zwischen den gegenüberliegenden parallel angeordneten Messfenstern (MF1) und (MF2) anzupassen, diesen auf die gewünschte Größe einzustellen und die exakte Position zu Bestimmen.

Gemäß der Erfindung ist eines der beiden Messfenster (MF1) oder (MF2), bevorzugt (MF2), mit einem axial beweglichen Stößel (ST), verbunden, der eine individuelle Einstellung, bevorzugt eine stufenlose individuelle Einstellung, des Abstands zwischen den beiden parallel angeordneten, einander gegenüberliegenden Messfenstern (MF1) und (MF2) erlaubt. Jedenfalls kann durch Bewegen des Stößels (ST), bevorzugt durch axiales Bewegen des Stößels (ST), der Abstand zwischen den beiden parallel angeordneten, einander gegenüberliegenden Messfenstern (MF1) und (MF2) vergrößert und verringert werden, wodurch der Messspalt (MS) und damit auch die Schichtstärke der, bevorzugt strömenden, zu vermessenden Probe individuell, bevorzugt stufenlos, angepasst werden kann.

Gemäß der Erfindung erlaubt der Stößel (ST) eine individuelle Einstellung, bevorzugt eine stufenlose individuelle Einstellung, des Abstands zwischen den beiden parallel angeordneten, einander gegenüberliegenden Messfenstern (MF1) und (MF2) erlaubt, wobei der Durchmesser (D) des mit dem Stößel verbundenen Messfenster (MF1) oder (MF2), bevorzugt (MF2), geringer ist als die Breite (d) der Grundfläche der Messzelle (MZ) (Vgl. FIG. 2a-d und 3a), an der das mit dem Stößel verbundene Messfenster (MF1) oder (MF2), bevorzugt (MF2), anliegt. Dadurch, dass der Durchmesser (D) des Messfensters (und konsequenter Weise des Stößels (ST)) geringer ist als die Breite (d) der Grundfläche der Messzelle (MZ), ist der Probenkanal beim zusammenfahren der Messfenster (MF1) und (MF2), also bei der Reduktion des Messspalts (MS), wobei der Stößel (ST) in die Messzelle vorgeschoben wird, nicht völlig verschlossen, es werden zwei Kanäle (K) seitlich des Stößels (ST) (links und rechts in Strömungsrichtung) ausgebildet. Der sich hieraus ergebende Vorteil ist ein ungehindertes entweichen der überschüssigen Probe über die Seiten des Messfensters in den Kanälen (K) beim verringern des Abstands zwischen den Messfenstern (MF1) und (MF2). Ein weiterer Vorteil dieser bevorzugten Anordnung der Messzelle (MZ) ist, dass ein Teil der in die Messzelle hineingeleiteten Probe, bevorzugt ein Teil der in die Messzelle hineingeleiteten strömenden Probe, den bei der Reduktion des Messspalts (MS) in die Messzelle hineinragenden Stößel (ST) ungehindert über die Kanäle (K) umfließen kann, wodurch dieser nicht vermessene Teil der Probe (die Probe hat den Messspalt (MS) nicht zwischen den Messfenstern (MF1) und (MF2) passiert), bevorzugt der nicht vermessene Teil der strömenden Probe, die Messzelle (MZ) nahezu ungehindert passieren kann (Vgl. FIG. 2a bis 2d und 3a-b). Eine solche Ausführung ist vor allem für den kontinuierlichen Messbetrieb oder die kontinuierliche Probenzufuhr geeignet, in dem nur bei Bedarf der Messspalt verringert wird, ohne den Durchfluss durch die Messzelle (MZ) selber signifikant zu reduzieren. In einem kontinuierlichen Messbetrieb muss die Zufuhr der Probe, bevorzugt der strömenden Probe, daher nicht reduziert, angehalten oder auf sonstiger Art unterbrochen werden.

Die Position eines Stößels (ST), und damit auch der Abstand der beiden Messfenster (MF1) und (MF2), kann im Allgemeinen durch ein Positioniersystem, bevorzugt durch einen oder mehrere Aktoren (AK) mit entsprechendem Controller (C-AK), eingestellt werden, wobei die genau erreichte Position durch ein Positionsmesssystem (POS) und einem entsprechenden Controller (C-POS) bestimmt werden kann. Beispielhafte Anordnungen sind in den Figuren 1a bis 1d sowie Figuren 2a bis 2d dargestellt.

In dem erfindungsgemäßen Sensor ist ein Messspalt (MS), das heißt der definierte Abstand der Messfenster (MF1) und (MF2), bevorzugt mit einer Genauigkeit von im Mittel 40 bis 100 nm, besonders bevorzugt von im Mittel 40 bis 60 nm, (Standardabweichung der Differenz des Ist- und Soll-Wertes), bevorzugt im durchströmten Betrieb, variabel einstellbar.

In dem erfindungsgemäßen Sensor ist eines der beiden Messfenster (MF1) oder (MF2), bevorzugt Messfenster (MF2), mit einem axial beweglichen Stößel (ST), verbunden, wobei die Position des Stößels (ST), und damit auch der Abstand zwischen den beiden Messfenstern (MF1) und (MF2), durch ein Positioniersystem, bevorzugt durch einen oder mehrere Aktoren (AK) mit entsprechendem Controller (C-AK), mit einer Genauigkeit von im Mittel 40 bis 100 nm, besonders bevorzugt von im Mittel 40 bis 60 nm, (Standardabweichung der Differenz des Ist- und Soll-Wertes), bevorzugt im durchströmten Betrieb, variabel eingestellt werden, wobei die genau erreichte Position bevorzugt durch ein Positionsmesssystem (POS) und einem entsprechenden Controller (C-POS) bestimmt werden kann. Beispielhafte Anordnungen sind in den Figuren 1a bis 1d sowie Figuren 2a bis 2d dargestellt.

In dem Fall, dass die zu messende Probe einen Messspalt (MS) durchströmt, tritt bei der Durchquerung des Spalts eine Scherung der Probe auf. Die Scherung wird bevorzugt dadurch erzielt, dass der Druckverlust in dem Spalt bevorzugt 0,1 bis 3 bar auf 1 bis 30 mm Länge, besonders bevorzugt 0,5 bis 1,5 bar auf 2 bis 15 mm Länge beträgt.

Um einen definierten Probenzustand aufrecht zu erhalten und damit vergleichbare Messdaten zu erzielen, ist - in dem Fall, dass die zu messende Probe einen Messspalt (MS) durchströmt - eine konstante Scherung der Probe bevorzugt. Diese wird bevorzugt durch kontinuierliche Überwachung des Eingangsdrucks, das heißt des Drucks an der Eingangsstelle der flüssigen Pigmentpräparation in dem Messspalt (MS) realisiert.

Die Drucküberwachung ist bevorzugt, um eine definierte Scherung am Messort zu ermöglichen. Wenn dies mit anderen Maßnahmen sichergestellt ist (z. B. bekannte Pumpleistung, Viskosität und Spaltweite), kann eine Druckmessung entfallen. Bei einer Druckmessung bieten sich mehrere Varianten an, nämlich die T-Konfiguration, die V-Konfiguration, eine Messung mit durchflossenem Druckaufnehmer, sowie eine Bohrung in der Produktzelle. Der Aufbau der genannten Konfigurationen ist dem Fachmann bekannt. Auswahlkriterium ist die hinreichend genaue Messung der relativ kleinen Drücke, die Unempfindlichkeit gegen Druckschwankungen (z. B. wenn das Produkt mit einer pulsierenden Pumpe gefördert wird), sowie die leichte Spülbarkeit (keine Toträume) oder zumindest Reinigbarkeit.

Die Einstellung des Eingangsdrucks ist unter anderem abhängig von dem Deckvermögen sowie von der Viskosität der Probe, insbesondere der flüssigen Pigmentpräparation, oder ihrer Verdünnung. Wird als Probe beispielsweise ein nicht stark deckender Lack eingesetzt, ist es erforderlich, eine Messzelle (MZ) mit einem größeren Messspalt (MS) zu wählen, als wenn ein stärker deckender Lack eingesetzt wird. Der Volumenstrom muss dann neu eingestellt werden, damit der Druckverlust konstant bleibt.

### d) mindestens eine Empfangsoptik (EO)(TM/VS/REM)

### Physikalische Funktion:

Das von der Probe ausgesendete Licht wird in einem Raumwinkelbereich durch die Empfangsoptik (EO) aufgenommen und zum Detektor (DET) geleitet. Je nachdem, auf welcher Seite dieser Raumwinkelbereich liegt, wird von Remission (gleiche Seite wie (LQ), bezogen auf das Messfenster) oder von Transmission/Vorwärtsstreuung (das durch die Probe auf die andere Seite der Lichtquelle (LQ), bezogen auf das Messfenster, hindurchgetretene Licht wird erfasst) gesprochen.

Die Empfangsoptik (EO) ist prinzipiell analog zur Beleuchtungsoptik (BO) aufgebaut (siehe Erläuterungen zu Beleuchtungsoptik (BO), Abschnitt b)). In einer Ausführungsform ist es möglich, eine winkelaufgelöste Messung des von der Probe ausgesendeten Lichtes zu machen, so dass mehrere, nur einen bestimmten Raumwinkelbereich erfassende Empfangsoptiken mit jeweiligem Detektor konzentrisch nebeneinander angeordnet und sequentiell oder besser parallel betrieben werden.

Bevorzugt sind die Empfangsoptiken (EO) unabhängig voneinander als faseroptisches System und/oder als konventionelles System mit Linsen und Blenden ausgeführt.

Üblicherweise enthält der erfindungsgemäße Sensor 1, 2, 3, 4, 5, 6, 7 oder 8 Empfangsoptiken (EO).

### e) mindestens einem Detektor (DET)(TM/VS/REM) zur Messung von durch Transmission erzeugten Transmissionssignalen, zur Messung von durch Vorwärtsstreuung erzeugten Vorwärtsstreusignalen beziehungsweise zur Messung von durch Remission erzeugten Remissionssignalen

### Physikalische Funktion:

Das von der Empfangsoptik (EO) gesammelte Licht gelangt zum Detektor (DET), der das Spektrum des Lichtes - im Allgemeinen im Vergleich zum Spektrum der Lichtquelle - analysiert. Dies geschieht in der Regel durch eine wellenlängenaufgelöste Messung der Intensität mit einem Spektrometer.

Der Detektor (DET) ist der komplementäre Gegenpart zur Lichtquelle (LQ). Die spektrale Eigenschaft der Probe wird durch Vergleich des beleuchtenden Spektrums (bevorzugt aus der Messung des Referenzkanals) mit dem von der Probe ausgestrahlten Spektrum bestimmt. Das Ergebnis ist stets ein Intensitätsverhältnis in einem Wellenlängenbereich, beziehungsweise eine Reihe von Messwerten (Array) für jeden der ermittelten Wellenlängenbereiche. Grundsätzlich ist es gleichwertig, ob die Wellenlängenselektion bereits lichtquellenseitig erfolgt (durchstimmbare Lichtquelle, oder wellenlängenselektives Element im Strahlengang), kombiniert mit einem breitbandigen Detektor mit einem Kanal, oder aber detektorseitig, zum Beispiel mit einem sogenannten monolitischen Spektrometer (Glasblock mit Dispersionsgitter und CCDbeziehnugsweise Diodenzeile). Letztere Ausführung benötigt keine bewegten Teile und ist schnell und robust.

Bevorzugt sind die Detektoren (DET) somit Intensitätsdetektoren, besonders bevorzugt spektral aufgelöste Intensitätsdetektoren, ganz besonders bevorzugt faseroptische monolithische Diodenzeilensensoren.

Das Auslesen der Empfänger und die Digitalisierung der Signale mit einer guten Auflösung (zum Beispiel 15 bit) sind kommerziell verfügbar, das Signal/Rausch-Verhältnis kann im Allgemeinen durch Mess-Wiederholung und Mittelung verbessert werden.

Üblicherweise enthält der erfindungsgemäße Sensor 2, 3, 4, 5, 6, 7 oder 8 Detektoren.

In einer bevorzugten Anordnung des erfindungsgemäßen Sensors sind mindestens 3 Detektoren (DET), bevorzugt 5 Detektoren (DET), zur mehrwinkligen Messung von durch Remission erzeugten Remissionssignalen unter mindestens 3 Raumwinkeln, bevorzugt 5 Raumwinkeln, im Raumwinkelbereich von 10° bis 115°, bevorzugt 15°, 25°, 45°, 75°, und 110°, zum Glanzwinkel der Lichtquelle (LQ)(REM) angeordnet.

In einer weiteren bevorzugten Anordnung des erfindungsgemäßen Sensors sind mindestens 2 Detektoren (DET), bevorzugt mindestens 3 Detektoren (DET), zur mehrwinkligen Messung von durch Vorwärtsstreuung erzeugten Vorwärtsstreusignalen unter mindestens 2 Raumwinkeln, bevorzugt mindestens 3 Raumwinkeln, im Raumwinkelbereich von >0° bis <90°, bevorzugt im Raumwinkelbereich von 10° bis 80°, besonders bevorzugt 20°, 30°, 45° und 65°, zur Lichtquelle (LQ)(VS) angeordnet.

In einer besonders bevorzugten Anordnung des erfindungsgemäßen Sensors sind mindestens 3 Detektoren (DET), bevorzugt 5 Detektoren (DET), zur mehrwinkligen Messung von durch Remission erzeugten Remissionssignalen unter mindestens 3 Raumwinkeln, bevorzugt 5 Raumwinkeln, im Raumwinkelbereich von 10° bis 115°, bevorzugt 15°, 25°, 45°, 75°, und 110°, zum Glanzwinkel der Lichtquelle (LQ)(REM) angeordnet, wobei diese Detektoren ebenfalls zur mehrwinkligen Detektion von durch Vorwärtsstreuung erzeugten Vorwärtsstreusignalen unter mindestens 2 Raumwinkeln, bevorzugt mindestens 3 Raumwinkeln, im Raumwinkelbereich von >0° bis <90°, bevorzugt im Raumwinkelbereich von 10° bis 80°, besonders bevorzugt 20°, 30°, 45° und 65°, zur Lichtquelle (LQ)(VS) verwendet werden.

Die Erfassung eines Raumwinkels unterliegt dabei einer bestimmten Toleranz, das heißt, das kein scharf begrenzter Raumwinkel sondern vielmehr ein Raumwinkelbereich erfasst werden kann, der Abhängig vom Raumwinkel unterschiedlich groß sein darf. Erlaubt sind zum Beispiel bei REM Messungen Toleranzen von bis zu ± 8° bei den Raumwinkeln 15°, 25° und 45° sowie bis zu ± 10° bei einem Raumwinkel von 75° und bis zu ± 20° bei einem Raumwinkel von 110°, jeweils zum Glanzwinkel der Lichtquelle (ASTM E2194).

Eine mehrwinklige Messung der Vorwärtsstreuung, insbesondere der Weißlichtstreuung, erlaubt eine umfassende Charakterisierung von winkelabhängigen Partikeln, insbesondere von Effektpigmenten oder Effektpigmentpräparationen, durch Bestimmen der Größe und Isotropie der Partikel sowie Erfassung der winkelabhängigen Farborte.

Wie bereits unter b) erwähnt, weist der Sensor bevorzugt zusätzlich zu der oder den Beleuchtungsoptiken (BO), eine oder mehrere Beleuchtungsoptiken, bevorzugt zwei Beleuchtungsoptiken als Referenzoptiken (RO), bevorzugt (RO)(TM/VS) und (RO)(REM), auf, und zusätzlich zu dem oder den Detektoren (DET)(TM/REM/VS) mindestens einen Detektor, bevorzugt zwei Detektoren, als Referenzdetektoren (RDET), bevorzugt (RDET)(TM/VS) und (RDET)(REM).

In einer bevorzugten Ausführungsform des erfindungsgemäßen Sensors liegen mehrere Lichtquellen (LQ) und mehrere Beleuchtungsoptiken (BO) vor, wobei eine Lichtquelle (LQ)(REM), eine Beleuchtungsoptik (BO)(REM), mindestens eine Empfangsoptik (EO)(TM/VS/REM) und mindestens ein Detektor (DET)(TM/VS/REM), auf der Seite des Messfensters (MF1), und eine Lichtquelle (LQ)(TM/VS) sowie eine Beleuchtungsoptik (BO)(TM/VS) auf der Seite des gegenüberliegenden Messfensters (MF2), angeordnet sind.

In einer weiteren bevorzugten Ausführungsform des erfindungsgemäßen Sensors liegen nur eine Lichtquelle (LQ) und nur eine Beleuchtungsoptik (BO) vor, die entweder auf der Seite des Messfensters (MF1) oder auf der Seite des Messfensters (MF2) angeordnet sind,
wobei bevorzugt mindestens eine Empfangsoptik (EO)(TM/VS) und mindestens ein Detektor (DET)(TM/VS) zur Messung der Transmission und/oder Vorwärtsstreuung an dem jeweils von der einen Lichtquelle (LQ) und einen Beleuchtungsoptik (BO) gegenüberliegenden Messfenster (MF2) oder (MF1) angeordnet sind, und,
bevorzugt mindestens eine Empfangsoptik (EO)(REM) und mindestens ein Detektor (DET)(REM) zur Messung der Remission an der Seite des Messfensters (MF1) oder (MF2) angeordnet sind, die der Seite der einen Lichtquelle (LQ) entspricht.

In einer besonders bevorzugten Ausführungsform des erfindungsgemäßen Sensors sind alle Einheiten des Sensors (a) bis (e) in einem gemeinsamen oder zweiteiligen Gehäuse untergebracht. Bevorzugt handelt es sich um ein mobiles Gehäuse, das ohne Schwierigkeiten zum Einsatzort transportiert werden kann, zum Beispiel ein Gehäuse auf Rollen. Bevorzugt wird das Gehäuse temperiert, da eine konstante Temperatur zu einer Verbesserung der Messgenauigkeit führt (Ventilation, thermostatgeregelte Wärmeabfuhr, Kühlwasser, Kühler/Lüfter und/oder thermostatisierte Messumgebung). Unabhängig davon kann es auch erforderlich sein, bei der Probentemperatur gewisse Toleranzen einzuhalten, da starkes Verdunsten der Lösungsmittel, eine Wärmeempfindlichkeit und thermochromatische Effekte möglich sind (Ventilation, thermostatgeregelte Wärmeabfuhr, Kühlwasser, Kühler/Lüfter und/oder thermostatisierte Messumgebung). Gleichzeitig werden thermische Wechselbelastungen vermieden, die zu mechanischen Veränderungen führen können. Des Weiteren wird durch das Gehäuse eine Berührung der Lichtwellenleiter sowie der anderen Elemente des Sensors vermieden und Lichtdichtheit gewährleistet. Durch das gemeinsame Gehäuse wird somit eine Erhöhung der Messgenauigkeit des Sensors erzielt.

In einer weiteren bevorzugten Ausführungsform enthält der erfindungsgemäße Sensor ein, wie in den Zeichnungen abgebildetes, Dichtsystem (DS(2)), der im Allgemeinen ein präzises Verstellen des Stößels (ST) mit den zur Verfügung stehenden Kräften bei Vorhandensein von feinteiligen Pigmenten, Polymeren und Lösemitteln ermöglicht. Die dem Fachmann bekannten Dichtsysteme sind nach diesen Gesichtspunkten auszuwählen und zu verwenden.

### Genauigkeit

Für die zuverlässige Erfassung von spektrometrischen Messdaten zur Ermittlung der farblichen Eigenschaften wie Farbort, Deckvermögen und Farbstärke, beziehungsweise Absorption (K oder A) und Streuung (S), flüssiger Proben ist es besonders wichtig, den Abstand der Messfenster (MF1) und (MF2) (das heißt den Messspalt (MS) und somit die Schichtstärke) genau einzustellen beziehungsweise zu kennen. Bevorzugt erfolgt die Einstellung des gewünschten Messspalts (MS) (Soll-Wert) mit einer Genauigkeit (Standardabweichung Ist-Soll-Wert) von im Mittel 40 bis 100 nm, besonders bevorzugt von im Mittel 40 bis 60 nm.

### Einschrittfahrweise

Bei der Einschrittfahrweise wird lediglich eine Schichtstärke eingestellt, deren absolute Kenntnis für die Ermittlung der Farbeigenschaften einer Probe möglichst genau bestimmt werden muss.

### Zweischrittfahrweise

Eine absolute Kenntnis der Schichtstärke ist nicht zwingend erforderlich, wenn zwei Messungen mit einer bekannten Differenz der Schichtstärke durchgeführt werden können und die Differenz der Transmission (dem Fachmann ist damit auch die ihm bekannte Extinktion zugänglich) dann auf diese Schichtstärkendifferenz bezogen wird. Diese Vorgehensweise erlaubt es die gleichen Produkteigenschaften wie bei der "Einschrittmessung" zu bestimmen. Die Zweischrittmessung erleichtert jedoch die Messung in der Praxis erheblich, da die technisch verfügbare absolute Genauigkeit von Piezoantrieben, welche bevorzugt als Aktoren (AK) eingesetzt werden, sehr hoch ist, während die Reproduzierbarkeit eines Messspalts (MS), das heißt der Schichtstärke, nach Demontage und Zusammenbau, zum Beispiel nach einer Reinigung der Messzelle im geöffneten Zustand, normalerweise eine Größenordnung schlechter ist. Wenn also in der Folge von Schichtstärke die Rede sein wird, ist stets die Möglichkeit angesprochen, zwei Messungen mit einer bekannten Schichtstärkendifferenz durchzuführen und auszuwerten.

### Bestimmung der idealen Schichtstärke

Zur vollständigen Charakterisierung der Probe ist es notwendig eine oder mehrere geeignete Schichtstärken einzustellen, wobei die für die Messung optimale Schichtstärke auch von der Probe selbst abhängig ist. Gemäß der Kubelka-Munk-Theorie können für REM Messungen eine deckende und eine nicht-deckende Schicht eingestellt werden und es ist für die TM und VS Messung wichtig, dass eine ausreichende Signalstärke vorhanden ist. Für die Messung der Vorwärtsstreuung bedeutet dies zum Beispiel, dass bei einer zu geringen Schichtstärke der Messeffekt, also die Änderung des Messsignals in Abhängigkeit der Interaktion mit der Probe, zu niedrig ausfällt, um die Eigenschaften der Probe eines Produktes mit genügender Präzision bestimmen zu können. Ist andererseits die Schichtstärke zu hoch gewählt, dringt so wenig Licht durch die Schicht, dass eine Messung dieser Lichtmenge nicht mit der erforderlichen Präzision möglich ist. Für alle drei Messarten, also für die Remission, Vorwärtsstreuung und Transmission, kann es daher erforderlich sein, unterschiedliche Schichtstärken einzustellen um den gewünschten signifikanten Messeffekt zu erzielen.

Der erfindungsgemäße Sensor zeichnet sich durch eine hochgenaue optische Geometrie für die quasi-simultane Messung der Transmission und/oder Vorwärtsstreuung und/oder Remission und zur simultanen Messung der Transmission und Vorwärtsstreuung, oder Transmission und Remission sowie ein hochgenaues System zum Einstellen eines Messspalts (MS) aus, wobei bevorzugt eines der beiden Messfenster (MF1) oder (MF2), bevorzugt (MF2), mit einem beweglichen Stößel (ST), bevorzugt einen axial beweglichen Stößel (ST), verbunden ist, der eine individuelle Einstellung, bevorzugt eine stufenlose individuelle Einstellung, des Abstands zwischen den beiden parallel angeordneten, einander gegenüberliegenden Messfenstern (MF1) und (MF2) erlaubt. Die Lichtquelle (LQ) oder die Lichtquellen (LQ) sind bevorzugt referenziert über einen Referenz-Detektor (RDET). Jedenfalls kann durch Bewegen des Stößels (ST), bevorzugt durch axiales Bewegen des Stößels (ST), der Abstand zwischen den beiden parallel angeordneten, einander gegenüberliegenden Messfenstern (MF1) und (MF2) vergrößert und verringert werden, wodurch der Messspalt (MS) und damit auch die Schichtstärke der, bevorzugt strömenden, Probe individuell, bevorzugt stufenlos, angepasst werden kann. Offensichtlich ist das mit dem Stößel (ST) verbundene Messfenster (MF1) oder (MF2), bevorzugt (MF2), auf einer Weise mit dem Stößel (ST) verbunden, die nicht zu einer Unterbrechung des Lichtstrahls durch die Probe führt.

Die üblicherweise bevorzugten Schichtstärken, einzustellen über den Messspalt (MS), liegen im Allgemeinen im Bereich von nicht deckenden (zum Beispiel 10-50 µm) bis zu deckenden Schichten (zum Beispiel 500 - 700 µm) für die Remissionsmessung und im Bereich von semitransparenten Schichten (10 - 300 µm) für die Transmissionsmessung und Vorwärtsstreumessung. Die Genauigkeit ist insbesondere bei der Transmissionsmessung eine Herausforderung, da der Fehler der Schichtstärke direkt in die damit eingestellte Konzentration der Pigmente eingeht, das heißt 1 µm Unsicherheit bei zum Beispiel einem Messspalt von 10 µm bedeuten +/- 10% Unsicherheit bei der Bestimmung der Pigmentkonzentration. Es wurde gefunden, dass bei einem definierten Abstand der Messfenster (MF1) und (MF2) von bevorzugt 1 bis 10000 µm, besonders bevorzugt 5 bis 5000 µm, ganz besonders bevorzugt 10 bis 500 µm geeignete Messergebnisse mit dem erfindungsgemäßen Sensor erhalten werden.

Die bevorzugte Referenzierung der Remissions-, Transmissions- und Vorwärtsstreumessung erfolgt im Allgemeinen dadurch, dass während der Messung sowohl das Probensignal als auch das Lichtquellensignal simultan spektral erfasst werden, so dass Schwankungen der Lichtquelle erfasst und aus den Probensignalen herausgerechnet werden können und damit die Remission, Transmission und Vorwärtsstreuung beleuchtungsunabhängig bestimmt werden können.

Mit Hilfe des erfindungsgemäßen Sensors konnten sehr hohe absolute Messgenauigkeiten von im Allgemeinen < 0,5 bis zu 0,05 ΔE erzielt werden, was durch eine absolute Messgenauigkeit von < 0,1% bis zu 0,01% der Rohmessdaten (Remissions-, Transmissions-, Vorwärtsstreuintensitäten) erreicht wird. Dabei handelt es sich bei ΔE um den für den Fachmann bekannten Begriff aus dem L*a*b*-Farbraum (CIELAB, EN ISO 11664-4) der den euklidischen Farbabstand der L*a*b*-Werte zweier Farborte angibt.

Vor Beginn der Messungen wird der Sensor bevorzugt kalibriert. Dies kann grundsätzlich auf jede beliebige, dem Fachmann bekannte Art und Weise erfolgen. Eine Auswahl geeigneter Kalbrierroutinen ist nachstehend genannt:

### Kalibrierroutinen

### a. Distanzpositioniersystem

Eine Kalibration kann erfolgen mittels absorbierenden (farbigen) Lösungen durch Einstellen verschiedener Messspalte (MS), Messen der zugehörigen Transmissionen und Auswerten nach dem für den Fachmann bekannten Gesetz nach Lambert Beer, in dem die Abschwächung der Intensität einer Strahlung bei dem Durchgang durch ein Medium mit einer absorbierenden Substanz, in Abhängigkeit von der Konzentration der absorbierenden Substanz und der Schichtdicke beschrieben wird. Besonders im linearen Bereich des Lambert-Beerschen-Gesetzes werden hohe Genauigkeiten bei der Kalibrierung des Positioniersystems erzielt.

### b. Remission

Eine Kalibrierung kann erfolgen mittels eines Weißstandards in fester oder flüssiger Form. Bevorzugt ist der Luftspalt zwischen dem für die Remissionsmessung relevanten Messfenster (MF1) beziehungsweise (MF2) und einem festen Weißstandard mit Immersionflüssigkeit, zum Beispiel Immersionsöl, gefüllt.

### c. Transmission

Eine Kalibrierung kann erfolgen mit einer klaren nichtabsorbierenden oder absorbierenden Lösung bei verschiedenen Messspalten (MS).

### d. Vorwärtsstreuung

Eine Kalibrierung der Vorwärtsstreuung ist mit flüssigen Streustandards (zum Beispiel Latex Dispersionen, TiO₂ Dispersionen, aber auch Emulsionen oder anderen Streustandards) bei einem oder mehreren Messspalten (MS) möglich.

### Verfahren

Die vorliegende Erfindung betrifft in einer weiteren Ausführungsform ein Verfahren zur quasi-simultanen Messung der Transmission und/oder Vorwärtsstreuung und/oder Remission und zur simultanen Messung der Transmission und Vorwärtsstreuung, oder Transmission und Remission einer Probe.

Der Messvorgang (Messverfahren) mit Hilfe des erfindungsgemäßen Sensors erfolgt bevorzugt durch Durchführung der folgenden Schritte i) bis viii) und beliebig häufige Wiederholungen, wobei die jeweiligen Schritte in Abhängigkeit von der gewünschten Verfahrensvariante durchgeführt werden, wobei die folgenden Verfahrensvarianten umfasst sind:

### Quasi-simultane Messvarianten:

a) Transmission und Vorwärtsstreuung
b) Transmission und Remission
c) Transmission und Vorwärtsstreuung und Remission
d) Vorwärtsstreuung und Remission

### Simultane Messvarianten:

e) Transmission und Vorwärtsstreuung
f) Transmission und Remission

### Verfahrensschritte:

i) Messspalt (MS) mit einem definierten Abstand X der Messfenster (MF1) und (MF2) einstellen
ii) Transmission (TM) messen, bei den Verfahrensvarianten a), b) und c),
iii) Vorwärtsstreuung (VS) messen, bei den Verfahrensvarianten a), c) und d),
iv) Remission (REM) messen, bei den Verfahrensvarianten b), c) und d)
v) Neuen, von Schritt i) abweichenden Messspalt (MS) mit einem definierten Abstand Y der Messfenster (MF1) und (MF2) einstellen,
vi) Transmission (TM) messen, bei den Verfahrensvarianten a), b) und c),
vii) Vorwärtsstreuung (VS) messen, bei den Verfahrensvarianten a), c) und d),
viii) Remission (REM) messen, bei den Verfahrensvarianten b), c) und d)
wobei die einzelnen Schritte i) bis viii) beliebig häufig Wiederholt werden können und
wobei die definierten Abstände X und Y unabhängig voneinander von 1 bis 10000 µm, bevorzugt von 5 bis 5000 µm, besonders bevorzugt von 10 bis 500 µm, betragen, wobei X ≠ Y.

Bei der quasi-simultanen Messvariante werden die nach Schritt i) beziehungsweise v) "Messspalt (MS) einstellen" erfolgenden Schritte ii) bis iv) beziehungsweise vi) bis viii) sequentiell innerhalb von 1 ms bis 10 s durchgeführt, wobei die Messvarianten TM/VS/REM, das heißt die Schritte ii) bis iv) beziehungsweise vi) bis viii), in beliebiger Reihenfolge (TM/VS/REM, TM/REM/VS, VS/TM/REM, VS/REM/TM, REM/TM/VS, und REM/VS/TM) oder in beliebiger Kombination einzelner Messschritte (TM/RM, TM/VS, VS/TM, VS/REM, REM/TM, REM/VS) durchgeführt werden können.

Bei der simultanen Messvariante erfolgen die Schritte "Transmission (TM) messen" und "Vorwärtsstreuung (VS) messen" oder "Transmission (TM) messen" und "Remission (REM) messen" simultan, das heißt zur gleichen Zeit.

Abhängig von der Ausführungsform des erfindungsgemäßen Sensors betrifft die vorliegende Erfindung daher ein Verfahren zur quasi-simultanen Messung der Transmission und/oder Vorwärtsstreuung und/oder Remission und zur simultanen Messung der Transmission und Vorwärtsstreuung, oder Transmission und Remission einer Probe mit einem erfindungsgemäßen Sensor, umfassend:
i) Ausbildung eines Messvolumens mit definierter Dicke (Schichtstärke) durch Einstellung des Abstands der Messfenster (MF1) und (MF2) zueinander, des Messspalts (MS) also, auf einen definierten Abstand von 1 bis 10000 µm, bevorzugt 5 bis 5000 µm, besonders bevorzugt 10 bis 500 µm, durch axiales Bewegen de Stößels (ST),
ii) Bestrahlung einer Probe unter einem oder mehreren Winkeln mit von einer Lichtquelle (LQ)(REM) ausgesandter elektromagnetischer Strahlung, wobei die elektromagnetische Strahlung mit der Probe in Wechselwirkung tritt und ein Teil der Strahlung nach Wechselwirkung mit der Probe diffus und/oder gerichtet reflektiert wird, und
   Durchstrahlung der Probe mit von einer Lichtquelle (LQ)(TM/VS) ausgesandter elektromagnetischer Strahlung, wobei die elektromagnetische Strahlung mit der Probe in Wechselwirkung tritt und ein Teil der Strahlung nach Wechselwirkung mit der Probe die Probe durchstrahlt beziehungsweise in Vorwärtsrichtung gestreut wird,
iii) Empfangen und Erfassen der diffus reflektierten Strahlung als Remissionssignal (Rückstreuung) unter einem oder mehreren Winkeln, und
   Empfangen und Erfassen der die Probe durchstrahlenden Strahlung als Vorwärtsstreuung unter einem oder mehreren Winkeln und/oder als Transmissionssignal (Transmission) unter einem Winkel.

Besonders bevorzugt umfasst das Verfahren zusätzlich die folgenden Schritte
iv) Empfangen und Erfassen eines Referenzsignals, wobei das Referenzsignal von derselben Lichtquelle (LQ)(REM), die zur Bestrahlung der Probe dient, ausgesandte elektromagnetische Strahlung ist, die nicht mit der Probe in Wechselwirkung tritt, und/oder
Empfangen und Erfassen eines Referenzsignals, wobei das Referenzsignal von derselben Lichtquelle (LQ)(TM/VS), die zur Durchstrahlung der Probe dient, ausgesandte elektromagnetische Strahlung ist, die nicht mit der Probe in Wechselwirkung tritt,
wobei das Remissionssignal und das Referenzsignal der (LQ)(REM) simultan erfasst werden, und
wobei das Transmissionssignal/Vorwärtsstreusignal und das Referenzsignal der (LQ)(TM/VS) simultan erfasst werden.

Damit wird erreicht, dass alle Signale, das heißt die Remissionssignale und das Referenzsignal von den gleichen zufälligen Schwankungen betroffen sind. Dies wird bevorzugt durch Verwendung von faseroptischen monolithischen Diodenzeilen-Spektrometern erreicht, die bevorzugt eine Auflösung von wenigstens 15 bit ermöglichen und die mit Integrationszeiten zwischen 4 ms und 6000 ms an die vorhandene Helligkeit angepasst werden. Die mit solchen Diodenzeilen-Spektrometern gemessenen Werte beziehen sich auf eine Diodennummer und müssen auf feste Wellenlängen interpoliert werden. Diese Interpolation ist besonders genau, wenn ein Spline verwendet wird, was bevorzugt ist.

In einer bevorzugten Ausführungsform ist das erfindungsgemäße Verfahren ein kontinuierliches Verfahren zur quasi-simultanen Messung der Transmission und/oder Vorwärtsstreuung und/oder Remission und zur simultanen Messung der Transmission und Vorwärtsstreuung, oder Transmission und Remission einer strömenden Probe, in dem nur bei Bedarf der Messspalt verringert wird, ohne den Durchfluss durch die Messzelle (MZ) selber signifikant zu reduzieren. In einem kontinuierlichen Messbetrieb muss die Zufuhr der strömenden Probe also nicht unterbrochen werden. Bevorzugt wird ein kontinuierliches Verfahren mit dem erfindungsgemäßen Sensor erreicht, in dem der Durchmesser (D) des mit dem Stößel verbundenen Messfenster (MF1) oder (MF2), bevorzugt (MF2), geringer ist als die Breite (d) der Grundfläche der Messzelle (MZ) (Vgl. FIG. 2a-d und 3a-b), an der das mit dem Stößel verbundene Messfenster (MF1) oder (MF2), bevorzugt (MF2), anliegt. Dadurch, dass der Durchmesser (D) des Messfensters (und konsequenter Weise des Stößels (ST)) geringer ist als die Breite (d) der Grundfläche der Messzelle (MZ), ist der Probenkanal beim zusammenfahren der Messfenster (MF1) und (MF2), also bei der Reduktion des Messspalts (MS), wobei der Stößel (ST) in die Messzelle vorgeschoben wird, nicht völlig verschlossen, sondern es werden zwei Kanäle (K) in Strömungsrichtung links und rechts vom Stößel (ST) ausgebildet, die den ungehinderten Durchgang eines Teils der in die Messzelle (MZ) eingeleiteten strömenden Probe erlauben, wodurch lediglich ein Teil der strömenden Probe in den Messspalt (MS) zwischen den Messfenstern (MF1) und (MF2) fließt und zur Messung zur Verfügung steht.

Mit Hilfe des erfindungsgemäßen Sensors sowie des erfindungsgemäßen Verfahrens ist eine genaue und schnelle Bestimmung der Farbeigenschaften (zum Beispiel Deckvermögen, Farbstärke, Farbort, winkelabhängiger Farbort) einer Probe, zum Beispiel von Anstrichmitteln wie Lacken und Farben, Pasten und Pigmenten und/oder ihrer stabilen Verdünnungen möglich.

### Verwendung

In einer weiteren Ausführungsform betrifft die vorliegende Erfindung die Verwendung des erfindungsgemäßen Sensors zur quasi-simultanen Messung der Transmission und/oder Vorwärtsstreuung und/oder Remission und zur simultanen Messung der Transmission und Vorwärtsstreuung, oder Transmission und Remission einer Probe zur Bestimmung der Farbeigenschaften von Anstrichmitteln wie Lacken und Farben, Pasten und Pigmenten oder ihren Verdünnungen.

Bevorzugt wird der erfindungsgemäße Sensor in einer beliebigen Verfahrensstufe bei der Herstellung, Weiterverarbeitung und Anwendung von Proben in Form von flüssigen Pigmentpräparationen und ihren Verdünnungen eingesetzt, bevorzugt zur Kontrolle der Pigmentmengen in Anstrichfarben, Lacken und Pigmentpasten; zur Qualitätskontrolle bei der Dispergierung von pigmentierten Anstrichfarben, Lacken und Pigmentpasten; zur Qualitätsbeurteilung bei der Herstellung von Anstrichfarben, bei der Lackherstellung und bei der Herstellung von Pigmentpasten; zur Steuerung einer Dosieranlage bei der Fertigung von Lacken, Anstrichfarben und Pigmentpasten durch Mischen verschiedener Flüssigkeiten/Suspensionen/Emulsionen; zur automatisch geregelten Farbeinstellung durch Tönen bei der Produktion von Anstrichfarben; bei der Lackproduktion und bei der Produktion von Pigmentpasten, zur Farbanpassung der Farbe des Lacks in einer Lackieranlage, die eine Dosieranlage für Farbpasten aufweist und/oder zur Kontrolle nachträglicher Farbänderungen durch Alterung oder Scherbeanspruchung von pigmentierten Anstrichfarben, Lacken oder Pigmentpasten; oder zur Überprüfung des optimalen Mahlgrads transparenter und semi-transparenter Pigmentpasten. Insbesondere eignet sich der erfindungsgemäße Sensor zur Messung von Bautenfarben und von Basislacken und Klarlacken im automobilen oder OEM-Bereich.

## Patentansprüche

1. Sensor zur quasi-simultanen Messung von mindestens zwei der drei Messarten Transmission (TM) und/oder Vorwärtsstreuung (VS) und/oder Remission (REM) und zur simultanen Messung der Transmission (TM) und Vorwärtsstreuung (VS), oder Transmission (TM) und Remission (REM) einer flüssigen Probe, wobei der Sensor aufgebaut ist aus:
a) eine oder mehrere Lichtquellen (LQ), und
b) eine oder mehrere Beleuchtungsoptiken (BO), und
c) mindestens eine Messzelle (MZ), und
d) 2, 3, 4, 5, 6, 7, oder 8 Empfangsoptiken (EO)(TM/VS/REM), und
e) 2, 3, 4, 5, 6, 7, oder 8 Detektoren (DET)(TM/VS/REM) zur Messung von durch Transmission erzeugten Transmissionssignalen, zur Messung von durch Vorwärtsstreuung erzeugten Vorwärtsstreusignalen beziehungsweise zur Messung von durch Remission erzeugten Remissionssignalen,
wobei die Messzelle (MZ) eine durchströmbare Zelle ist, die zwei gegenüberliegende parallel angeordnete Messfenster (MF1) und (MF2) aufweist, die so zueinander angeordnet sind, dass ein Messspalt (MS) zwischen den Messfenstern gebildet wird, der von der zu messenden Probe ausgefüllt wird, wobei die Messfenster einen definierten Abstand zueinander aufweisen der im durchströmten Betrieb variabel einstellbar ist, wobei die Messzelle derart ausgestaltet ist, dass eines der beiden Messfenster (MF1,MF2) mit einem axial beweglichen Stößel (ST), verbunden ist, und
wobei die Position des Stößels (ST), und damit auch der definierte Abstand der Messfenster (MF1) und (MF2) im durchströmten Betrieb durch ein Positioniersystem variabel mit einer Genauigkeit von im Mittel 40 bis 100 nm als Standardabweichung der Differenz des Ist-und Soll-Wertes, einstellbar ist.

2. Sensor nach Anspruch 1, **dadurch gekennzeichnet, dass** bei der quasi-simultanen Messung mindestens zwei Messungen ausgewählt aus Transmission (TM), Vorwärtsstreuung (VS) und Remission (REM) in Kombination durchgeführt werden.

3. Sensor nach Anspruch 1 oder 2, **dadurch gekennzeichnet, dass** die durch Vorwärtsstreuung erzeugten Vorwärtsstreusignale gleichzeitig unter mindestens 2 Raumwinkeln, bevorzugt mindestens 3 Raumwinkeln, im Raumwinkelbereich von >0° bis <90°, bevorzugt im Raumwinkelbereich von 10° bis 80°, besonders bevorzugt 20°, 30° und 65°, zur Lichtquelle (LQ)(VS) gemessen werden.

4. Sensor nach einem der Ansprüche 1 bis 3, **dadurch gekennzeichnet, dass** die durch Remission erzeugten Remissionssignale gleichzeitig unter mindestens 3 Raumwinkeln, bevorzugt 5 Raumwinkeln, im Raumwinkelbereich von 10° bis 115°, bevorzugt 15°, 25°, 45°, 75°, und 110°, zum Glanzwinkel der Lichtquelle (LQ)(REM) gemessen werden.

5. Sensor nach einem der Ansprüche 1 bis 4, **dadurch gekennzeichnet**, die Position des Stößels (ST), durch einen oder mehrere Aktoren (AK) mit entsprechendem Controller (C-AK), eingestellt wird, wobei die genau erreichte Position durch ein Positionsmesssystem (POS) und einem entsprechenden Controller (C-POS) bestimmt werden kann.

6. Sensor nach einem der Ansprüche 1 bis 5, **dadurch gekennzeichnet, dass** der Durchmesser (D) des mit dem Stößel (ST) verbundenen Messfensters (MF1) oder (MF2), bevorzugt (MF2), geringer ist als die Breite (d) der Grundfläche der Messzelle (MZ), an der das mit dem Stößel verbundene Messfenster (MF1) beziehungsweise (MF2), bevorzugt (MF2), anliegt.

7. Sensor nach einem der Ansprüche 1 bis 6, **dadurch gekennzeichnet, dass** der definierte Abstand der Messfenster (MF1) und (MF2) 1 bis 10000 µm, bevorzugt 5 bis 5000 µm, besonders bevorzugt 10 bis 500 µm beträgt.

8. Sensor nach einem der Ansprüche 1 bis 7, **dadurch gekennzeichnet, dass** die Empfangsoptiken unabhängig voneinander als faseroptisches System und/oder als konventionelles System mit Linsen und Blenden ausgeführt sind.

9. Sensor nach einem der Ansprüche 1 bis 8, **dadurch gekennzeichnet, dass** die Detektoren Intensitätsdetektoren, bevorzugt spektral aufgelöste Intensitätsdetektoren, besonders bevorzugt faseroptische monolithische Diodenzeilensensoren sind.

10. Sensor nach einem der Ansprüche 1 bis 9, **dadurch gekennzeichnet, dass** alle Einheiten des Sensors (a) bis (e) in einem gemeinsamen Gehäuse untergebracht sind, in dem eine Ventilation und eine thermostatgeregelte Wärmeabfuhr erfolgt.

11. Verfahren zur quasi-simultanen Messung von mindestens zwei der drei Messarten Transmission (TM) und/oder Vorwärtsstreuung (VS) und/oder Remission (REM) und zur simultanen Messung der Transmission (TM) und Vorwärtsstreuung (VS), oder Transmission (TM) und Remission (REM) einer flüssigen Probe mit einem Sensor gemäß einem der Ansprüche 1 bis 10, umfassend:
i) Ausbildung eines Messvolumens mit definierter Dicke durch Einstellung des Abstands der Messfenster (MF1) und (MF2) zueinander auf einen definierten Abstand von 1 bis 10000 µm, bevorzugt 5 bis 5000 µm, besonders bevorzugt 10 bis 500 µm, durch axiales Bewegen des Stößels durch den Positioniersystem.
ii) Bestrahlung einer Probe unter einem oder mehreren Winkeln mit von der Lichtquelle (LQ)(REM) ausgesandter elektromagnetischer Strahlung, wobei die elektromagnetische Strahlung mit der Probe in Wechselwirkung tritt und ein Teil der Strahlung nach Wechselwirkung mit der Probe diffus und/oder gerichtet reflektiert wird, und
Durchstrahlung der Probe mit von der Lichtquelle (LQ)(TM/VS) ausgesandter elektromagnetischer Strahlung, wobei die elektromagnetische Strahlung mit der Probe in Wechselwirkung tritt und ein Teil der Strahlung nach Wechselwirkung mit der Probe die Probe durchstrahlt beziehungsweise in Vorwärtsrichtung gestreut wird,
iii) Empfangen und Erfassen der diffus reflektierten Strahlung als Remissionssignal unter einem oder mehreren Winkeln, und
Empfangen und Erfassen der die Probe durchdringenden Strahlung als Transmissionssignal unter einem Winkel und/oder als Vorwärtsstreusignal unter einem oder mehreren Winkeln; wobei alternativ nur das Transmissionssignal unter einem Winkel simultan mit dem Vorwärtsstreusignal oder dem Remissionssignal unter einem oder mehreren Winkeln gemessen werden kann.

12. Verfahren nach Anspruch 11, umfassend zusätzlich den folgenden Schritt:
iv) Empfangen und Erfassen eines Referenzsignals, wobei das Referenzsignal von derselben Lichtquelle (LQ)(REM), die zur Bestrahlung der Probe dient, ausgesandte elektromagnetische Strahlung ist, die nicht mit der Probe in Wechselwirkung tritt und/oder
Empfangen und Erfassen eines Referenzsignals, wobei das Referenzsignal von derselben Lichtquelle (LQ)(TM/VS), die zur Durchstrahlung der Probe dient, ausgesandte elektromagnetische Strahlung ist, die nicht mit der Probe in Wechselwirkung tritt,
wobei das Remissionssignal und das Referenzsignal der (LQ)(REM) simultan erfasst werden, und
wobei das Transmissionssignal/Vorwärtsstreusignal und das Referenzsignal der (LQ)(TM/VS) simultan erfasst werden.

13. Verfahren nach Anspruch 11 oder 12, **dadurch gekennzeichnet, dass** es sich um ein kontinuierliches Verfahren handelt, bei dem lediglich ein Teil der in die Messzelle (MZ) eingeleiteten Probe zwischen den Messfenstern (MF1) und (MF2) strömt und der andere Teil, der in die Messzelle (MZ) eingeleiteten Probe, ungehindert aus der Messzelle (MZ), bevorzugt über aus dem Messfenster (MF1), Stößel (ST) sowie Messzelle (MZ) gebildeten Kanäle (K) in Strömungsrichtung links und rechts vom Stößel (ST), entweichen kann.

14. Verwendung des Sensors nach einem der Ansprüche 1 bis 10 zur quasi-simultanen Messung von mindestens zwei der drei Messarten Transmission (TM) und/oder Vorwärtsstreuung (VS) und/oder Remission (REM) und zur simultanen Messung der Transmission (TM) und Vorwärtsstreuung (VS), oder Transmission (TM) und Remission (REM) einer flüssigen Probe.

15. Verwendung nach Anspruch 14 in einer beliebigen Verfahrensstufe bei der Herstellung, Weiterverarbeitung und Anwendung von Proben in Form von flüssigen Pigmentpräparationen oder ihren stabilen Verdünnungen, bevorzugt zur Kontrolle der Pigmentmengen in Anstrichfarben, Lacken und Pigmentpasten; zur Qualitätskontrolle bei der Dispergierung von pigmentierten Anstrichfarben, Lacken und Pigmentpasten; zur Qualitätsbeurteilung bei der Herstellung von Anstrichfarben, bei der Lackherstellung und bei der Herstellung von Pigmentpasten; zur Steuerung einer Dosieranlage bei der Fertigung von Lacken, Anstrichfarben und Pigmentpasten durch Mischen verschiedener Flüssigkeiten/Suspensionen/Emulsionen; zur automatisch geregelten Farbeinstellung durch Tönen bei der Produktion von Anstrichfarben; bei der Lackproduktion und bei der Produktion von Pigmentpasten, zur Farbanpassung der Farbe des Lacks in einer Lackieranlage, die eine Dosieranlage für Farbpasten aufweist und/oder zur Kontrolle nachträglicher Farbänderungen durch Alterung oder Scherbeanspruchung von pigmentierten Anstrichfarben, Lacken oder Pigmentpasten; oder zur Überprüfung des optimalen Mahlgrads transparenter und semi-transparenter Pigmentpasten.

## Claims

1. Sensor for quasi-simultaneous measurement of at least two of the three measurement types of transmission (TM) and/or forward scattering (VS) and/or diffuse reflection (REM) and for simultaneous measurement of the transmission (TM) and forward scattering (VS) or the transmission (TM) and diffuse reflection (REM) of a liquid sample, wherein the sensor is constructed from:
a) one or more light sources (LQ) and
b) one or more illumination optical units (BO) and
c) at least one measurement cell (MZ) and
d) 2, 3, 4, 5, 6, 7 or 8 reception optical units (EO) (TM/VS/REM) and
e) 2, 3, 4, 5, 6, 7 or 8 detectors (DET) (TM/VS/REM) for measuring transmission signals generated by transmission, for measuring forward scattering signals generated by forward scattering and for measuring diffuse reflection signals generated by diffuse reflection,
wherein the measurement cell (MZ) is a flow cell, which has two opposing measurement windows (MF1) and (MF2) disposed in parallel, which are disposed relative to one another such that a measurement gap (MS) is formed between the measurement windows and filled by the sample to be measured, wherein the measurement windows have a defined distance from one another, which is variably adjustable during flow operation, wherein the measurement cell is configured in such a way that one of the two measurement windows (MF1, MF2) is connected to an axially movable plunger (ST) and wherein the position of the plunger (ST), and hence also the defined distance between the measurement windows (MF1) and (MF2) is variably adjustable by a positioning system during the flow operation, with an accuracy of on average 40 to 100 nm as a standard deviation of the difference between the actual value and target value.

2. Sensor according to Claim 1, **characterized in that** at least two measurements selected from transmission (TM), forward scattering (VS) and diffuse reflection (REM) are carried out in combination within the scope of the quasi-simultaneous measurement.

3. Sensor according to Claim 1 or 2, **characterized in that** the forward scattering signals generated by forward scattering are measured simultaneously from at least 2 solid angles, preferably from at least 3 solid angles, in the solid angle range from >0° to <90°, preferably in the solid angle range from 10° to 80°, particularly preferably 20°, 30° and 65° with respect to the light source (LQ) (VS).

4. Sensor according to any one of Claims 1 to 3, **characterized in that** the diffuse reflection signals generated by the diffuse reflection are measured simultaneously from at least 3 solid angles, preferably 5 solid angles, in the solid angle range from 10° to 115°, preferably 15°, 25°, 45°, 75° and 110°, with respect to the glancing angle of the light source (LQ) (REM).

5. Sensor according to any one of Claims 1 to 4, **characterized in that** the position of the plunger (ST) is set by one or more actuators (AK) with a corresponding controller (C-AK), wherein the precisely reached position can be determined by a position measurement system (POS) and a corresponding controller (C-POS).

6. Sensor according to any one of Claims 1 to 5, **characterized in that** the diameter (D) of the measurement window (MF1) or (MF2), preferably (MF2), connected to the plunger (ST) is less than the width (d) of the base area of the measurement cell (MZ) against which the measurement window (MF1) or (MF2), preferably (MF2), connected to the plunger abuts.

7. Sensor according to any one of Claims 1 to 6, **characterized in that** the defined distance between the measurement windows (MF1) and (MF2) is 1 to 10 000 µm, preferably 5 to 5000 µm, particularly preferably 10 to 500 µm.

8. Sensor according to any one of Claims 1 to 7, **characterized in that** the reception optical units are embodied independently of one another as a fibre-optic system and/or a conventional system with lenses and stops.

9. Sensor according to any one of Claims 1 to 8, **characterized in that** the detectors are intensity detectors, preferably spectrally resolved intensity detectors, particularly preferably fibre-optic monolithic diode line sensors.

10. Sensor according to any one of Claims 1 to 9, **characterized in that** all units of the sensor (a) to (e) are housed in a common housing, in which there is ventilation and thermostat-regulated heat dissipation.

11. Process for quasi-simultaneous measurement of at least two of the three measurement types of transmission (TM) and/or forward scattering (VS) and/or diffuse reflection (REM) and for simultaneous measurement of the transmission (TM) and forward scattering (VS) or the transmission (TM) and diffuse reflection (REM) of a liquid sample using a sensor according to any one of Claims 1 to 10, said process including:
i) forming a measurement volume with a defined thickness by setting distance between the measurement windows (MF1) and (MF2) with respect to one another to a defined distance ranging from 1 to 10 000 µm, preferably 5 to 5000 µm, particularly preferably 10 to 500 µm, by axially moving the plunger by way of the positioning system,
ii) irradiating the sample from one or more angles using electromagnetic radiation emitted by the light source (LQ) (REM), wherein the electromagnetic radiation interacts with the sample and some of the radiation is reflected in diffuse and/or specular fashion following the interaction with the sample, and trans-illuminating the sample with electromagnetic radiation emitted by the light source (LQ) (TM/VS), where the electromagnetic radiation interacts with the sample and some of the radiation radiates through the sample or is scattered in the forward direction following interaction with the sample,
iii) receiving and capturing the diffusely reflected radiation as diffuse reflection signal from one or more angles, and
receiving and capturing the radiation passing through the sample as transmission signal from one angle and/or as forward scattering signal from one or more angles;
wherein, alternatively, only the transmission signal from one angle can be measured simultaneously with the forward scattering signal or the diffuse reflection signal from one or more angles.

12. Process according to Claim 11, additionally including the following step:
iv) receiving and capturing a reference signal, wherein the reference signal is electromagnetic radiation emitted by the same light source (LQ) (REM) that serves to irradiate the sample, said electromagnetic radiation not interacting with the sample and/or
receiving and capturing a reference signal, wherein the reference signal is electromagnetic radiation emitted by the same light source (LQ) (TM/VS) that serves to trans-illuminate the sample, said electromagnetic radiation not interacting with the sample, wherein the diffuse reflection signal and the reference signal of the (LQ) (REM) are captured simultaneously, and
wherein the transmission signal/forward scattering signal and the reference signal of the (LQ) (TM/VS) are captured simultaneously.

13. Process according to Claim 11 or 12, **characterized in that** this is a continuous process, in which only some of the sample introduced into the measurement cell (MZ) flows between the measurement windows (MF1) and (MF2) and the rest of the sample introduced into the measurement cell (MZ) can escape from the measurement cell (MZ) unimpeded, preferably via channels (K) to the left and right of the plunger (ST) in the flow direction, said channels being formed by the measurement window (MF1), plunger (ST) and measurement cell (MZ).

14. Use of the sensor according to any one of Claims 1 to 10 for quasi-simultaneous measurement of at least two of the three measurement types of transmission (TM) and/or forward scattering (VS) and/or diffuse reflection (REM) and for simultaneous measurement of the transmission (TM) and forward scattering (VS) or the transmission (TM) and diffuse reflection (REM) of a liquid sample.

15. Use according to Claim 14 within any process stage during the production, further processing and application of samples in the form of liquid pigment preparations or their stable thinners, preferably for controlling the pigment amounts in paint colours, coatings and pigment pastes; for controlling the quality when dispersing pigmented paint colours, coatings and pigment pastes; for assessing the quality when producing paint colours, when producing coatings and when producing pigment pastes; for controlling a metering unit when manufacturing coatings, paint colours and pigment pastes by mixing various liquids/suspensions/emulsions; for automatically regulated setting of the colour by tinting during the production of paint colours; during the coating production and during the production of pigment pastes, for colour adaptation of the colour of the coating in a coating unit, which has a metering unit for colour pastes and/or for monitoring subsequent colour changes by ageing or shear loads of pigmented paint colours, coatings or pigment pastes; or for monitoring the optimal degree of grinding of transparent and semi-transparent pigment pastes.

## Revendications

1. Capteur pour mesurer quasi simultanément au moins deux de trois types de mesure comprenant la transmittance (TM) et/ou la diffusion vers l'avant (VS) et/ou la luminance de réflexion (REM) et pour mesurer simultanément la transmittance (TM) et la diffusion vers l'avant (VS), ou la transmittance (TM) et la luminance de réflexion (REM) d'un échantillon liquide, dans lequel le capteur se compose :
a) d'une ou de plusieurs sources lumineuses (LQ), et
b) d'une ou de plusieurs optiques d'éclairage (BO), et
c) d'au moins une cellule de mesure (MZ), et
d) de 2, 3, 4, 5, 6, 7 ou 8 optiques de réception (EO) (TM/VS/REM), et
e) de 2, 3, 4, 5, 6, 7 ou 8 détecteurs (DET) (TM/VS/REM) pour mesurer des signaux de transmittance générés par transmittance, pour mesurer des signaux de diffusion vers l'avant générés par diffusion vers l'avant ou pour mesurer des signaux de luminance de réflexion générés par luminance de réflexion,
dans lequel la cellule de mesure (MZ) est une cellule à circulation qui comporte deux fenêtres de mesure opposées (MF1) et (MF2) disposées parallèlement, qui sont disposées l'une par rapport à l'autre de telle manière qu'il se forme entre les fenêtres de mesure un interstice de mesure (MS) qui est rempli par l'échantillon à mesurer, dans lequel les fenêtres de mesure présentent entre elles une distance définie qui est réglable de manière variable en mode de circulation, dans lequel la cellule de mesure est conçue de telle manière que l'une des deux fenêtres de mesure (MF1, MF2) soit reliée à un piston mobile axialement (ST), et
dans lequel la position du piston (ST), et donc également la distance définie entre les fenêtres de mesure (MF1) et (MF2) en mode de circulation, peuvent être réglées de manière variable par un système de positionnement avec une précision allant en moyenne de 40 à 100 nm en tant qu'écart type de la différence entre la valeur réelle et la valeur de consigne.

2. Capteur selon la revendication 1, **caractérisé en ce que**, lors de la mesure quasi-simultanée, au moins deux mesures choisies parmi la transmittance (TM), la diffusion vers l'avant (VS) et la luminance de réflexion (REM) sont effectuées de manière combinée.

3. Capteur selon la revendication 1 ou 2, **caractérisé en ce que** les signaux de diffusion vers l'avant générés par diffusion vers l'avant sont mesurés simultanément sous au moins 2 angles solides, de préférence au moins 3 angles solides, dans la plage d'angles solides de >0° à <90°, de préférence dans la plage d'angles solides de 10° à 80°, de manière particulièrement préférée de 20°, 30° et 65°, par rapport à la source lumineuse (LQ)(VS).

4. Capteur selon l'une des revendications 1 à 3, **caractérisé en ce que** les signaux de luminance de réflexion générés par luminance de réflexion sont mesurés simultanément sous au moins 3 angles solides, de préférence 5 angles solides, dans la plage d'angles solides de 10° à 115°, de préférence 15°, 25°, 45°, 75° et 110°, par rapport à l'angle de Bragg de la source lumineuse (LQ)(REM).

5. Capteur selon l'une des revendications 1 à 4, **caractérisé en ce que** la position du piston (ST) est réglée par un ou plusieurs actionneurs (AK) à l'aide d'un dispositif de commande correspondant (C-AK), dans lequel la position précise atteinte peut être déterminée par un système de mesure de position (POS) et un dispositif de commande correspondant (C-POS).

6. Capteur selon l'une des revendications 1 à 5, **caractérisé en ce que** le diamètre (D) de la fenêtre de mesure (MF1) ou (MF2), de préférence (MF2), reliée au piston (ST) est inférieur à la largeur (d) de la surface de base de la cellule de mesure (MZ) contre laquelle repose la fenêtre de mesure (MF1) ou (MF2), de préférence (MF2), reliée au piston.

7. Capteur selon l'une des revendications 1 à 6, **caractérisé en ce que** la distance définie entre les fenêtres de mesure (MF1) et (MF2) est de 1 à 10000 µm, de préférence de 5 à 5000 µm, et de manière particulièrement préférée de 10 à 500 µm.

8. Capteur selon l'une des revendications 1 à 7, **caractérisé en ce que** les optiques de réception sont réalisées indépendamment l'une de l'autre sous la forme d'un système à fibres optiques et/ou d'un système classique à lentilles et diaphragmes.

9. Capteur selon l'une des revendications 1 à 8, **caractérisé en ce que** les détecteurs sont des détecteurs d'intensité, de préférence des détecteurs d'intensité à résolution spectrale, et de manière particulièrement préférée des détecteurs à ligne de diodes monolithiques à fibres optiques.

10. Capteur selon l'une des revendications 1 à 9, **caractérisé en ce que** toutes les unités du capteur (a) à (e) sont logées dans un boîtier commun dans lequel ont lieu une ventilation et une dissipation thermique régulée par thermostat.

11. Procédé pour mesurer quasi simultanément au moins deux de trois types de mesure comprenant la transmittance (TM) et/ou la diffusion vers l'avant (VS) et/ou la luminance de réflexion (REM) et pour mesurer simultanément la transmittance (TM) et la diffusion vers l'avant (VS), ou la transmittance (TM) et la luminance de réflexion (REM) d'un échantillon liquide à l'aide d'un capteur selon l'une des revendications 1 à 10, comprenant :
i) la formation d'un volume de mesure d'épaisseur définie par réglage de la distance entre les fenêtres de mesure (MF1) et (MF2) à une distance définie de 1 à 10000 µm, de préférence de 5 à 5000 µm, et de manière particulièrement préférée de 10 à 500 µm, par déplacement axial du piston à travers le système de positionnement,
ii) l'exposition d'un échantillon sous un ou plusieurs angles au rayonnement électromagnétique émis par la source lumineuse (LQ) (REM), dans lequel le rayonnement électromagnétique interagit avec l'échantillon et une partie du rayonnement est réfléchie de manière diffuse et/ou directionnelle après interaction avec l'échantillon, et
l'irradiation de l'échantillon avec le rayonnement électromagnétique émis par la source lumineuse (LQ)(TM/VS), dans lequel le rayonnement électromagnétique interagit avec l'échantillon et une partie du rayonnement après interaction avec l'échantillon passe à travers l'échantillon ou est diffusé vers l'avant,
iii) la réception et la détection du rayonnement diffus réfléchi sous la forme d'un signal de luminance de réflexion sous un ou plusieurs angles, et
la réception et la détection du rayonnement pénétrant dans l'échantillon sous la forme d'un signal de transmittance sous un certain angle et/ou d'un signal de diffusion vers l'avant sous un ou plusieurs angles ; dans lequel, en variante, seul le signal de transmittance sous un certain angle peut être mesuré simultanément avec le signal de diffusion vers l'avant ou le signal de luminance de réflexion sous un ou plusieurs angles.

12. Procédé selon la revendication 11, comprenant en outre l'étape suivante :
iv) la réception et la détection d'un signal de référence, dans lequel le signal de référence est un rayonnement électromagnétique émis par la même source lumineuse (LQ)(REM) que celle utilisée pour l'exposition de l'échantillon, et qui n'interagit pas avec l'échantillon et/ou
la réception et la détection d'un signal de référence, dans lequel le signal de référence est un rayonnement électromagnétique émis par la même source lumineuse (LQ)(TM/VS) que celle utilisée pour l'irradiation de l'échantillon, et qui n'interagit pas avec l'échantillon, dans lequel le signal de luminance de réflexion et le signal de référence de la source lumineuse (LQ) (REM) sont détectés simultanément, et
dans lequel le signal de transmittance/diffusion vers l'avant et le signal de référence de la source lumineuse (LQ)(TM/VS) sont détectés simultanément.

13. Procédé selon la revendication 11 ou 12, **caractérisé en ce qu'**il s'agit d'un procédé continu dans lequel seule une partie de l'échantillon introduit dans la cellule de mesure (MZ) circule entre les fenêtres de mesure (MF1) et (MF2) et l'autre partie de l'échantillon introduit dans la cellule de mesure (MZ) peut s'échapper sans restriction de la cellule de mesure (MZ), de préférence par le biais de canaux (K) formés par la fenêtre de mesure (MF1), le piston (ST) et la cellule de mesure (MZ) à gauche et à droite du piston (ST) dans le sens de l'écoulement.

14. Utilisation du capteur selon l'une des revendications 1 à 10 pour mesurer quasi simultanément au moins deux de trois types de mesure comprenant la transmittance (TM) et/ou la diffusion vers l'avant (VS) et/ou la luminance de réflexion (REM) et pour mesurer simultanément la transmittance (TM) et la diffusion vers l'avant (VS), ou la transmittance (TM) et la luminance de réflexion (REM), d'un échantillon liquide.

15. Utilisation selon la revendication 14 à un stade quelconque du procédé lors de la production, du post-traitement et de l'utilisation d'échantillons sous la forme de préparations pigmentaires liquides ou de leurs dilutions stables, de préférence pour le contrôle des quantités de pigment dans les peintures, les vernis et les pâtes pigmentaires pigmentés ; pour le contrôle de qualité lors de la dispersion de peintures, de vernis et de pâtes pigmentaires pigmentés ; pour l'évaluation de la qualité lors de la production de peintures, lors de l'application de vernis et lors de la production de pâtes pigmentaires ; pour la commande d'une installation de dosage lors de la fabrication de peintures, de vernis et de pâtes pigmentaires par mélange de différents liquides/suspensions/émulsions ; pour le réglage automatique de la couleur par mise en teinte lors de la production de peintures ; lors de la production de vernis et lors de la production de pâtes pigmentaires, pour l'adaptation de la couleur du vernis dans une installation d'application de vernis qui comporte une unité de dosage pour pâtes colorantes et/ou pour le contrôle de variations de couleurs ultérieures dues au vieillissement ou à la contrainte de cisaillement de peintures, de vernis ou pâtes pigmentaires pigmentés ; ou pour la vérification du degré optimal de broyage de pâtes pigmentaires transparentes et semi-transparentes.
